# EUROPÄISCHE PATENTANMELDUNG

(11) **EP 4 586 270 A1**
(43) Veröffentlichungstag der Anmeldung: **16.07.2025**
(21) Anmeldenummer: 25150120.1
(22) Anmeldetag: 02.01.2025
(51) Int. Cl.: G16H 40/63, G16H 50/70

(54) **MEDIZINISCHES SYSTEM, TRAGBARE ANZEIGEN, VERFAHREN ZUM BETRIEB EINES MEDIZINISCHEN SYSTEMS UND/ODER EINER TRAGBAREN ANZEIGE UND VERFAHREN**

(30) Priorität: 12.01.2024 DE 102024100941
(71) Anmelder: KARL STORZ SE & Co. KG, 78532 Baden-Württemberg Tuttlingen (DE)
(72) Erfinder: BÜHLER, Clemens, 78532 Tuttlingen (DE); GERLACH, Irina, 78532 Tuttlingen (DE)

(57) **Zusammenfassung**

Die vorliegende Erfindung betrifft ein medizinisches System (100) umfassend eine Patientenüberwachungseinheit (102), die dazu eingerichtet ist, einen Vitalparameter eines Patienten (126) zu erfassen, eine tragbare Anzeige (104), die an einem Kopf eines Benutzers (124) tragbar ist und die in einem getragenen Zustand einem Benutzer (124) eine zumindest im Wesentlichen unbeeinträchtigte Betrachtung einer Umgebung (106) in einem Sichtfeld (108) des Benutzers (124) gestattet, wobei die tragbare Anzeige (104) dazu eingerichtet ist, in dem getragenen Zustand eine Vitalinformationseinblendung (110) in dem Sichtfeld (108) des Benutzers (124) einzublenden, die auf dem einen Vitalparameter beruht, und eine Patientenzustandserkennungseinheit (140), die dazu eingerichtet ist, zumindest einen Patientenzustandsparameter zu ermitteln, der sich auf einen aktuellen Vitalzustand des Patienten (126) bezieht, wobei die tragbare Anzeige (104) dazu eingerichtet ist, eine Einblendung der Vitalinformationseinblendung (110) in Abhängigkeit des Patientenzustandsparameters vorzunehmen oder zu unterlassen.

## Beschreibung

Die vorliegende Anmeldung betrifft ein medizinisches System, tragbare Anzeigen, ein Verfahren zum Betrieb eines medizinischen Systems und/oder einer tragbaren Anzeige und ein Verfahren.

Medizinisches Fachpersonal wird heutzutage bei der Durchführung diagnostischer, therapeutischer und/oder operativer Prozeduren durch eine Vielzahl von Systemen unterstützt. Mit dem kontinuierlichen Fortschritt der Technologie und kostengünstigeren Fertigungsmethoden nimmt die Anzahl verfügbarer Systeme stetig zu. In einem Operationssaal kommen beispielsweise zahlreiche Geräte zum Einsatz, darunter Operationsmikroskope, Anästhesiegeräte, Beatmungsgeräte, chirurgische Instrumente, mobile Röntgengeräte, Ultraschallgeräte, Patientenüberwachungseinheiten, Navigationssysteme und viele weitere. Viele dieser Geräte verfügen über eigene Anzeigeeinheiten und stellen dem medizinischen Fachpersonal visuelle Informationen über den Patienten zur Verfügung. Ein behandelnder Chirurg muss etwa während einer Operation in zeitkritischen Situationen aus der Vielzahl an Anzeigeeinheiten diejenigen identifizieren, die im Moment relevant sind, und gleichzeitig aus einer großen Informationsmenge die gerade benötigten Informationen filtern.

Während des Eingriffs kann der Chirurg beispielsweise seinen Fokus und Blick auf eine Inzisionsstelle eines Patienten richten, um den Fortschritt des Eingriffs visuell zu überwachen und mögliche Komplikationen schnell zu erkennen. Benötigt er jedoch Informationen über den Vitalzustand des Patienten, muss er den Blick von der Inzisionsstelle abwenden und auf eine Anzeigevorrichtung richten, die die entsprechenden Informationen über den Vitalzustand bereitstellt. Dies führt zu einer höheren Belastung der Konzentration des Chirurgen, da er ständig den Fokus wechseln muss. Besonders bei komplexen Eingriffen, die über mehrere Stunden dauern können, kann dies schwerwiegende Folgen haben, Fehler können unterlaufen, und die Sicherheit des Eingriffs kann beeinträchtigt sein.

Zusätzlich kann sich der Zustand des Patienten während des Eingriffs plötzlich verschlechtern. Die Erfinder haben erkannt, dass gerade in solch zeitkritischen Situationen der Chirurg seine wesentliche Aufmerksamkeit dem Eingriff selbst widmen sollte. Wichtige Informationen über den Zustand des Patienten muss der behandelnde Chirurg jedoch nach dem Stand der Technik in kurzer Zeit aus der Vielzahl der Anzeigevorrichtungen herausfiltern und verarbeiten.

Gemäß einem weiteren Beispiel kann es während einer Intubation zur Herstellung und Aufrechterhaltung der Atemwege zu Komplikationen kommen. Bei einer Intubation etwa nach einem Unfall können Blut und Schwellungen die Intubation erheblich erschweren. Zudem kann sich der Patient in einem kritischen Zustand befinden. Informationen über den Zustand des Patienten können entscheidend für den Ausgang der Prozedur sein. Der Behandler könnte beispielsweise Informationen über die Sauerstoffsättigung des Bluts oder den Puls des Patienten benötigen. Wenn der Behandler diese Informationen jedoch in einer hektischen Situation aus einer Vielzahl von Anzeigeeinheiten gewinnen muss, kann sein Fokus vom eigentlichen Durchführen der Intubation abgelenkt werden, und die Qualität der Durchführung der Prozedur kann abnehmen.

Die Erfinder haben erkannt, dass ein Benutzer bei der Erlangung und Verarbeitung von Information über einen Patienten unterstützt werden sollte. Die Information sollte zielgerichteter und situationsgerechter bereitgestellt werden. Allgemein sollte der Benutzer entlastet werden, sodass er sich besser auf eine Versorgung des Patienten konzentrieren kann.

Ausgehend vom Stand der Technik liegt der Erfindung die Aufgabe zugrunde, Information über einen Patienten effizient bereitzustellen.

Die Aufgabe wird erfindungsgemäß durch ein medizinisches System, tragbare Anzeigen, ein Verfahren zum Betrieb eines medizinischen Systems und/oder einer tragbaren Anzeige und ein Verfahren gelöst, wie sie hierin beschrieben und in den Ansprüchen definiert sind. Zudem sind weitere Aspekte der Erfindung beschrieben, die die Erfindung erklären.

Gemäß einem Aspekt kann die vorliegende Erfindung vorsehen, ein medizinisches System bereitzustellen. Das medizinisches System umfasst eine Patientenüberwachungseinheit, die dazu eingerichtet ist, einen Vitalparameter eines Patienten zu erfassen, eine tragbare Anzeige, die an einem Kopf eines Benutzers tragbar ist und die in einem getragenen Zustand einem Benutzer eine zumindest im Wesentlichen unbeeinträchtigte Betrachtung einer Umgebung in einem Sichtfeld des Benutzers gestattet, wobei die tragbare Anzeige dazu eingerichtet ist, in dem getragenen Zustand eine Vitalinformationseinblendung in dem Sichtfeld des Benutzers einzublenden, die auf dem einen Vitalparameter beruht, und eine Patientenzustandserkennungseinheit, die dazu eingerichtet ist, zumindest einen Patientenzustandsparameter zu ermitteln, der sich auf einen aktuellen Vitalzustand des Patienten bezieht. Die tragbare Anzeige ist gemäß diesem Aspekt dazu eingerichtet, eine Einblendung der Vitalinformationseinblendung in Abhängigkeit des Patientenzustandsparameters vorzunehmen oder zu unterlassen.

Gemäß einem weiteren Aspekt sieht die vorliegende Erfindung vor, eine tragbare Anzeige für ein medizinisches System gemäß dem vorigen Aspekt bereitzustellen.

Gemäß einem weiteren Aspekt sieht die vorliegende Erfindung vor, eine tragbare Anzeige, insbesondere für ein medizinisches System gemäß einem der vorigen Aspekte, bereitzustellen. Die tragbare Anzeige ist an einem Kopf eines Benutzers tragbar und gestattet in einem getragenen Zustand einem Benutzer eine zumindest im Wesentlichen unbeeinträchtigte Betrachtung einer Umgebung in einem Sichtfeld des Benutzers gestattet. Die tragbare Anzeige ist dazu eingerichtet, in dem getragenen Zustand eine Vitalinformationseinblendung in dem Sichtfeld des Benutzers einzublenden, die auf dem einen Vitalparameter beruht. Zudem umfasst die tragbare Anzeige eine Patientenzustandserkennungseinheit, die dazu eingerichtet ist, zumindest einen Patientenzustandsparameter zu ermitteln, der sich auf einen aktuellen Vitalzustand des Patienten bezieht. Die tragbare Anzeige ist gemäß diesem Aspekt dazu eingerichtet, eine Einblendung der Vitalinformationseinblendung in Abhängigkeit des Patientenzustandsparameters vorzunehmen oder zu unterlassen.

Gemäß einem weiteren Aspekt sieht die vorliegende Erfindung vor, ein Verfahren zum Betrieb eines medizinischen Systems nach einem der vorigen Aspekte und/oder einer tragbaren Anzeige nach einem der vorigen Aspekte bereitzustellen.

Gemäß einem weiteren Aspekt sieht die vorliegende Erfindung vor, ein Verfahren, insbesondere durchgeführt mittels eines medizinischen Systems nach einem der vorigen Aspekte und/oder einer tragbaren Anzeige nach einem der vorigen Aspekte, bereitzustellen. Das Verfahren umfasst die Schritte eines Erfassens eines Vitalparameters eines Patienten, eines Bereitstellens einer tragbaren Anzeige für einen Benutzer, die an einem Kopf eines Benutzers tragbar ist und die in einem getragenen Zustand dem Benutzer eine zumindest im Wesentlichen unbeeinträchtigte Betrachtung einer Umgebung in einem Sichtfeld des Benutzers gestattet, eines Einblendens einer Vitalinformationseinblendung in dem Sichtfeld des Benutzers mittels der tragbaren Anzeige in einem getragenen Zustand der Anzeige, die auf dem Vitalparameter beruht, eines Ermittelns eines Patientenzustandsparameters, der sich auf einen aktuellen Vitalzustand des Patienten bezieht, und eines Vornehmens oder Unterlassens des Einblendens der Vitalinformationseinblendung in Abhängigkeit des Patientenzustandsparameters.

Durch diese Merkmale kann Information über einen Patienten effizient bereitgestellt werden. Die Information wird zielgerichteter, bedarfsgerechter und situationsspezifischer bereitgestellt. Die Vitalinformation wird dem Benutzer beispielsweise dann zur Verfügung gestellt, wenn sie im Zusammenhang mit dem Vitalzustand beachtenswert ist oder berücksichtigt werden sollte. Dem Benutzer kann insgesamt weniger Information bereitgestellt werden, die zu einem bestimmten Zeitpunkt bereitgestellte Information jedoch von vorrangiger Bedeutung sein. Die bereitgestellt Vitalinformation kann sich in Abhängigkeit vom Zustand des Patienten dynamisch ändern. Der Benutzer kann über diese Änderung, insbesondere situationsspezifisch, benachrichtigt werden, indem die entsprechende Vitalinformation eingeblendet wird. Dabei muss der Benutzer nicht von seinem Fokus auf eine gerade durchgeführte Prozedur ablassen, sondern bekommt die Vitalinformation in seinem Sichtfeld mittels der Vitalinformationseinblendung eingeblendet. Der Benutzer muss folglich eine geringere Menge bereitgestellter Information verarbeiten, muss nicht aufmerksam eine Änderung des Patientenzustands an verschiedenen Anzeigevorrichtungen beobachten und kann sich besser auf eine gerade durchzuführende Prozedur konzentrieren. Dadurch werden die Belastung und die Anforderung an die Konzentration und Fokussierung des Benutzers reduziert. Dadurch kann eine höhere Qualität in der Durchführung der Prozedur wie etwa einer Operation oder Intubation erreicht werden. Die Patientenversorgung kann verbessert und das Risiko von Behandlungsfehlern reduziert werden.

Gemäß einem weiteren Aspekt sieht die vorliegende Erfindung vor, ein medizinisches System bereitzustellen. Das medizinische System umfasst eine Patientenüberwachungseinheit, die dazu eingerichtet ist, einen Vitalparameter eines Patienten zu erfassen, eine tragbare Anzeige, die an einem Kopf eines Benutzers tragbar ist und die in einem getragenen Zustand dem Benutzer eine zumindest im Wesentlichen unbeeinträchtigte Betrachtung einer Umgebung in einem Sichtfeld des Benutzers gestattet, wobei die tragbare Anzeige dazu eingerichtet ist, in dem getragenen Zustand eine Vitalinformationseinblendung in dem Sichtfeld des Benutzers einzublenden, die auf dem Vitalparameter beruht, und eine Sichtfelderkennungseinheit, die dazu eingerichtet ist, zumindest einen Sichtfeldparameter zu ermitteln, der sich auf ein aktuelles Sichtfeld des Benutzers bezieht. Die tragbare Anzeige ist dazu eingerichtet, eine Einblendung der Vitalinformationseinblendung in Abhängigkeit des Sichtfeldparameters vorzunehmen oder zu unterlassen.

Gemäß einem weiteren Aspekt sieht die vorliegende Erfindung vor, eine tragbare Anzeige für ein medizinisches System gemäß dem vorigen Aspekt bereitzustellen.

Gemäß einem weiteren Aspekt sieht die vorliegende Erfindung vor, eine tragbare Anzeige, insbesondere für ein medizinisches System gemäß einem der vorigen Aspekte, bereitzustellen. Die tragbare Anzeige ist an einem Kopf eines Benutzers tragbar und gestattet in einem getragenen Zustand dem Benutzer eine zumindest im Wesentlichen unbeeinträchtigte Betrachtung einer Umgebung in einem Sichtfeld des Benutzers. Die tragbare Anzeige ist dazu eingerichtet, in dem getragenen Zustand eine Vitalinformationseinblendung in dem Sichtfeld des Benutzers einzublenden, die auf einem Vitalparameter beruht. Zudem umfasst die tragbare Anzeige eine Sichtfelderkennungseinheit, die dazu eingerichtet ist, zumindest einen Sichtfeldparameter zu ermitteln, der sich auf das aktuelle Sichtfeld des Benutzers bezieht. Ferner ist die tragbare Anzeige dazu eingerichtet, eine Einblendung der Vitalinformationseinblendung in Abhängigkeit des Sichtfeldparameters vorzunehmen und/oder zu unterlassen.

Gemäß einem weiteren Aspekt sieht die vorliegende Erfindung vor, ein Verfahren zum Betrieb eines medizinischen Systems nach einem der vorigen Aspekte und/oder einer tragbaren Anzeige nach einem der vorigen Aspekte bereitzustellen.

Gemäß einem weiteren Aspekt sieht die vorliegende Erfindung vor, ein Verfahren, insbesondere durchgeführt mittels eines medizinischen Systems nach einem der vorigen Aspekte und/oder einer tragbaren Anzeige nach einem der vorigen Aspekte, bereitzustellen. Das Verfahren umfasst die Schritte eines Erfassens eines Vitalparameters eines Patienten, eines Bereitstellens einer tragbaren Anzeige für einen Benutzer, die an einem Kopf eines Benutzers tragbar ist und die in einem getragenen Zustand dem Benutzer eine zumindest im Wesentlichen unbeeinträchtigte Betrachtung einer Umgebung in einem Sichtfeld des Benutzers gestattet, eines Einblendens einer Vitalinformationseinblendung in dem Sichtfeld des Benutzers mittels der tragbaren Anzeige in einem getragenen Zustand der Anzeige, die auf dem Vitalparameter beruht, eines Ermittelns eines Sichtfeldparameters, der sich auf ein aktuelle Sichtfeld des Benutzers bezieht, und eines Vornehmens oder Unterlassens des Einblendens der Vitalinformationseinblendung in Abhängigkeit des Sichtfeldparameters.

Durch diese Merkmale kann Information über einen Patienten effizient bereitgestellt werden. Die Information wird zielgerichteter, bedarfsgerechter und situationsspezifischer bereitgestellt. Dem Benutzer wird in Abhängigkeit des aktuellen Sichtfelds Information über den Patienten bereitgestellt. Das Sichtfeld wird erkannt und anhand des Sichtfelds die Vitalinformationseinblendung vorgenommen oder unterlassen. Beispielsweise kann erreicht werden, dass eine überflüssige und/oder redundante Informationsbereitstellung unterlassen wird. In Situationen, in denen das aktuelle Sichtfeld eine Vitalinformationseinblendung unnötig oder sogar nachteilig machen würde, kann die Einblendung unterlassen werden. Wiederrum kann sie in Situationen, in denen die Vitalinformationseinblendung unterstützend wirkt, vorgenommen werden. Es kann ein dynamisches Ein- und Ausblenden der Vitalinformation je nach dem aktuellen Sichtfeld vorgenommen werden. Der Benutzer wird also durch die Einblendung der Vitalinformation derart unterstützt, dass er sich besser auf eine Prozedur konzentrieren kann, die er gerade durchführt. Das Erfassen und Verarbeiten der Information wird erleichtert. Zudem muss der Benutzer nicht zwangsläufig zu der Patientenüberwachungseinheit blicken, um Vitalinformation über den Patienten zu erlangen. Diese wird in seinem Sichtfeld sichtfeldabhängig eingeblendet. Es wird folglich ein Komfort für den Benutzer erhöht und eine notwendige Aufmerksamkeit und/oder Fokussierung, um die Vitalinformation zu erlangen, reduziert. Dadurch kann eine höhere Qualität in der Durchführung der Prozedur wie etwa einer Operation oder Intubation erreicht werden. Die Patientenversorgung kann verbessert und das Risiko von Behandlungsfehlern reduziert werden.

Gemäß einem weiteren Aspekt kann die vorliegende Erfindung vorsehen, ein medizinisches System bereitzustellen. Das medizinisches System umfasst eine Patientenüberwachungseinheit, die dazu eingerichtet ist, einen Vitalparameter eines Patienten zu erfassen, eine tragbare Anzeige, die an einem Kopf eines Benutzers tragbar ist und die in einem getragenen Zustand einem Benutzer eine zumindest im Wesentlichen unbeeinträchtigte Betrachtung einer Umgebung in einem Sichtfeld des Benutzers gestattet, wobei die tragbare Anzeige dazu eingerichtet ist, in dem getragenen Zustand eine Vitalinformationseinblendung in dem Sichtfeld des Benutzers einzublenden, die auf dem einen Vitalparameter beruht, und eine Prozedurschritterkennungseinheit, die dazu eingerichtet ist, zumindest einen Prozedurschrittparameter zu ermitteln, der beschreibt, in welchem Schritt von mehreren Schritten einer vom Benutzer am Patienten durchzuführenden Prozedur sich der Benutzer aktuell befindet. Dabei ist die tragbare Anzeige dazu eingerichtet, eine Einblendung der Vitalinformationseinblendung in Abhängigkeit des Prozedurschrittparameters vorzunehmen und/oder zu unterlassen.

Gemäß einem weiteren Aspekt sieht die vorliegende Erfindung vor, eine tragbare Anzeige für ein medizinisches System gemäß dem vorigen Aspekt bereitzustellen.

Gemäß einem weiteren Aspekt sieht die vorliegende Erfindung vor, eine tragbare Anzeige, insbesondere für ein medizinisches System gemäß einem der vorigen Aspekte, bereitzustellen. Die tragbare Anzeige ist an einem Kopf eines Benutzers tragbar und gestattet in einem getragenen Zustand einem Benutzer eine zumindest im Wesentlichen unbeeinträchtigte Betrachtung einer Umgebung in einem Sichtfeld des Benutzers. Die tragbare Anzeige ist dazu eingerichtet, in dem getragenen Zustand eine Vitalinformationseinblendung in dem Sichtfeld des Benutzers einzublenden, die auf dem einen Vitalparameter beruht und umfasst eine Prozedurschritterkennungseinheit, die dazu eingerichtet ist, zumindest einen Prozedurschrittparameter zu ermitteln, der beschreibt, in welchem Schritt von mehreren Schritten einer vom Benutzer am Patienten durchzuführenden Prozedur sich der Benutzer aktuell befindet. Dabei ist die tragbare Anzeige dazu eingerichtet, eine Einblendung der Vitalinformationseinblendung in Abhängigkeit des Prozedurschrittparameters vorzunehmen und/oder zu unterlassen.

Gemäß einem weiteren Aspekt sieht die vorliegende Erfindung vor, ein Verfahren zum Betrieb eines medizinischen Systems nach einem der vorigen Aspekte und/oder einer tragbaren Anzeige nach einem der vorigen Aspekte bereitzustellen.

Gemäß einem weiteren Aspekt sieht die vorliegende Erfindung vor, ein Verfahren, insbesondere durchgeführt mittels eines medizinischen Systems nach einem der vorigen Aspekte und/oder einer tragbaren Anzeige nach einem der vorigen Aspekte, bereitzustellen. Das Verfahren umfasst die Schritte eines Erfassens eines Vitalparameters eines Patienten, eines Bereitstellens einer tragbaren Anzeige für einen Benutzer, die an einem Kopf eines Benutzers tragbar ist und die in einem getragenen Zustand dem Benutzer eine zumindest im Wesentlichen unbeeinträchtigte Betrachtung einer Umgebung in einem Sichtfeld des Benutzers gestattet, eines Einblendens einer Vitalinformationseinblendung in dem Sichtfeld des Benutzers mittels der tragbaren Anzeige in einem getragenen Zustand der Anzeige, die auf dem Vitalparameter beruht, eines Ermittelns eines Prozedurschrittparameter, der beschreibt, in welchem Schritt von mehreren Schritten einer vom Benutzer am Patienten durchzuführenden Prozedur sich der Benutzer aktuell befindet, und eines Vornehmens oder Unterlassens des Einblendens der Vitalinformationseinblendung in Abhängigkeit des Prozedurschrittparameters.

Durch diese Merkmale kann Information über einen Patienten effizient bereitgestellt werden. Die Information wird zielgerichteter, bedarfsgerechter und situationsspezifischer bereitgestellt. Der Benutzer bekommt zielgerichtet jene Vitalinformation bereitgestellt, die er im Zusammenhang mit einem bestimmten Prozedurschritt benötigt. Die gesamte zu einem bestimmten Zeitpunkt bereitgestellte Information kann reduziert werden und lediglich die vorrangig wichtige Information bereitgestellt werden. Der Benutzer selbst muss weniger Konzentration darauf verwenden, aus einer Menge an Information, die zu dem Zeitpunkt wichtige Information zu isolieren und/oder zu verarbeiten. Eine Belastung des Benutzers wird dadurch reduziert und dieser kann sich besser auf die durchzuführende Prozedur fokussieren. Der Benutzer müsste sich beispielsweise nach dem Stand der Technik bei jedem neuen Prozedurschritt auf eine neue Anzeigevorrichtung, auf der Vitalinformation bereitgestellt werden kann, konzentrieren und diese aus einer in seiner Umgebung angeordnete Anzeigevorrichtungen auswählen. Die Erfinder haben jedoch erkannt, dass der Benutzer entlastet werden kann, wenn die für den entsprechenden Schritt notwendige Information erkannt, vorausgewählt und dem Benutzer im Sichtfeld eingeblendet wird. Information von geringerer Wichtigkeit kann ausgeblendet werden, um eine Belastung des Benutzers zu reduzieren. Dadurch kann eine höhere Qualität in der Durchführung der Prozedur wie etwa einer Operation oder Intubation erreicht werden. Die Patientenversorgung kann verbessert und das Risiko von Behandlungsfehlern reduziert werden.

Durch die unterschiedlichen obengenannten Aspekte können Verbesserungen im Bereich der Bereitstellung von Vitalinformation eines Patienten ermöglicht werden.

Wenn ein System, eine Vorrichtung, eine Einheit und/oder eine Komponente im Zusammenhang mit verschiedenen Aspekten der vorliegenden Erfindung beschrieben ist, kann es sich grundsätzlich um dasselbe System, dieselbe Vorrichtung, dieselbe Einheit und/oder dieselbe Komponente handeln. Das kann beispielsweise bedeuten, dass ein medizinisches System Merkmale verschiedener hierin beschriebener Aspekte aufweisen kann. Ebenso kann eine tragbare Anzeige Merkmale verschiedener hierin beschriebener Aspekte aufweisen. In anderen Worten kann ein medizinisches System die Funktionen und damit einhergehenden Vorteile einiger und/oder aller medizinischen Systeme der hierin beschriebenen Aspekte in sich vereinen.

Das medizinische System kann mehrere Vorrichtungen, Einheiten und/oder Komponenten umfassen. Das medizinische System kann für eine mobile und/oder stationäre Anwendung vorgesehen sein. Beispielsweise kann das medizinische System in einem Rettungswagen angeordnet und/oder aufgebaut sein, in einem Notfallkoffer oder ähnlichem für einen Notfallmediziner zu einem Patienten tragbar und vor Ort bei diesem bereitstellbar sein, und/oder in einem Operationssaal, einem Behandlungszimmer vorgesehen sein. Ferner kann das medizinische System erweiterbar sein, beispielsweise indem weitere Patientenüberwachungseinheiten hinzugeschaltet werden und/oder eine bestehende Patientenüberwachungseinheit erweitert wird. Ferner kann das medizinische System zumindest teilweise in bereits bestehende Ausrüstung von Rettungswagen, Notfallkoffern, Operationssälen und/oder integriert werden und/oder zumindest teilweise durch diese ausgebildet sein. In diesem Sinne kann das medizinische System bereits bestehende Systeme in ihrer Funktion erweitern und/oder zusätzlich zu diesen Systemen eine weitere Funktionalität bereitstellen.

Vitalparameter können messbare physiologische Zustandsgrößen umfassen, die Aufschluss über den Zustand und/oder die Funktionen des Patienten geben. Vitalparameter können entscheidend für die Überwachung der vitalen Funktionen und Veränderungen dieser sein. Grundsätzlich erlauben Vitalparameter eine Einschätzung des Gesundheitszustands und/oder des Vitalzustands eines Patienten. Insbesondere im Zusammenhang mit einer Prozedur kann eine Überwachung der Vitalparameter entscheidend sein, da eine Veränderung dieser auf eine kritische Situation hinweisen kann. Ferner kann anhand einer Änderung eines Vitalparameters Kenntnis über eine Reaktion des Patienten auf eine Prozedur gewonnen werden. Wird der Patient beispielsweise intubiert, kann etwa anhand des Vitalparameters Sauerstoffsättigung ein Erfolg der Prozedur Intubation überprüft werden und/oder eingeschätzt werden, ob es zu einer kritischen Situation während der Prozedur kommen könnte. Es kann also etwa eingeschätzt werden, ob der Tubus richtig gelegt wurde. Beispiele für Vitalparameter sind die Herzfrequenz, die Atemfrequenz, der Blutdruck, die Körpertemperatur, die Sauerstoffsättigung und/oder Kohlendioxidsättigung im Blut, Kohlendioxidkonzentration in der Atemluft, elektrische Hirnaktivität, der intrakranielle Druck und viele weitere. Insbesondere kann es eine Vielzahl prozedurspezifischer Vitalparameter geben, etwa solche, die im Zusammenhang mit einer Prozedur von Bedeutung sind, die an der etwa an der Leber und/oder an dem Gehirn durchgeführt wird.

Eine Prozedur kann sich beispielsweise auf eine therapeutische, diagnostische und/oder chirurgische Maßnahme beziehen. In anderen Worten kann die Prozedur etwa einen chirurgischen Eingriff, eine endoskopische Untersuchung, lebensrettende Maßnahmen in der Notfallmedizin und/oder dergleichen umfassen.

Die Patientenüberwachungseinheit kann eines oder mehrere, insbesondere medizinische/s, Gerät/e und/oder System/e umfassen, die jeweils zur Erfassung von Vitalparametern und anderer wichtiger physiologischer Informationen eines Patienten eingerichtet sind. Insbesondere kann mittels der Patientenüberwachungseinheit zumindest ein Vitalparameter während der Prozedur erfasst werden. Dadurch kann mittels einer Patientenüberwachungseinheit zumindest im Wesentlichen in Echtzeit und/oder mit geringem zeitlichem Verzug der Zustand des Patienten überwacht werden. Eine Patientenüberwachungseinheit kann als mobiles, tragbares medizinisches Gerät und/oder System ausgebildet sein. Das kann bedeuten, dass ein Benutzer die Patientenüberwachungseinheit zum Patienten tragen kann und diese dort zur Erfassung der Vitalparameter einsetzbar ist. Ferner kann die Patientenüberwachungseinheit beispielsweise auch fest montiert in einem Operationsaal vorgesehen sein. Alternativ oder zusätzlich kann die Patientenüberwachungseinheit eine Gesamtheit an Geräten und/oder System umfassen, die zur Erfassung von Vitalparametern eingerichtet sind. Häufig umfasst eine Patientenüberwachungseinheit mehrere modular aufgebaute medizinische Geräte, die z. B. auf einem Geräteturm installiert sind und mittels dieses Geräteturms im Operationssaal verfahrbar sind. Weitere Module können der Patientenüberwachungseinheit bedarfsweise hinzugeschaltet werden. Beispiele für solche entsprechende Module, Geräte und/oder Systeme können ein EKG-Gerät, ein Pulsoximeter, ein Blutdruckmessgerät, ein Atemfrequenzmesser, ein Thermometer, ein intrakranieller Druckmonitor und/oder ein EEG-Gerät sein.

Die Patientenüberwachungseinheit kann mehrere Anzeigevorrichtungen umfassen, die jeweils lediglich einen Teil der Vitalparameter anzeigen, beziehungsweise auf denen eine Darstellung beruhend auf einem Vitalparameter anzeigbar sind. Etwa jedes Modul, Gerät und/oder System der Patientenüberwachungseinheit kann eine eigene Anzeigevorrichtung umfassen, auf der lediglich ein mittels des entsprechenden Moduls, Geräts und/oder Systems erfasster Vitalparameter anzeigbar ist. Der Benutzer muss also, um Information über einen bestimmten Vitalparameter zu erlangen, die richtige aus den mehreren Anzeigevorrichtungen auswählen. Dies kann insbesondere in hektischen Situationen, Notfällen und/oder dergleichen zu einem kritischen Zeitverlust und/oder einer höheren Belastung des Benutzers führen.

Ferner kann die Patientenüberwachungseinheit eine zentrale Anzeigevorrichtung umfassen, an der mehrere und/oder zumindest im Wesentlichen alle erfassten Vitalparameter anzeigbar sind. In anderen Worten kann erfasste Information der einzelnen Module, Geräte und/oder Systeme gemeinsam an der zentralen Anzeigevorrichtung verfügbar gemacht werden.

Wie bereits beschrieben kann das medizinische System beispielsweise in einem klinischen Umfeld in einem Operationssaal anwendbar sein. Das medizinische System kann jedoch auch in Notfallsituationen in der Intensivmedizin beispielsweise in einem Rettungswagen oder auch etwa an einem Unfallort zur Erstversorgung anwendbar sein. Der Benutzer kann in dem Zusammenhang medizinisches Fachpersonal, insbesondere Chirurgen, Anästhesisten, Notfallmediziner und/oder dergleichen umfassen.

Allgemein kann mittels der tragbaren Anzeige Information visuell für den Benutzer verfügbar gemacht werden. Dies kann beispielsweise durch eine Projektion einer Darstellung, insbesondere direkt auf die Netzhaut, erreicht werden. Ferner kann eine Darstellung auch auf einem Display anzeigbar sein. Das Display kann insbesondere durchsichtig sein. Das kann bedeuten, dass der Benutzer durch das Display hindurchsehen kann. Das Display kann etwa integral mit einem Brillenglas ausgebildet sein und/oder brillenglasartig in dem Stehstrahl des Benutzers angeordnet sein. Die tragbare Anzeige kann eine Virtual-Reality-Brille (VR-Brille), eine Augmented-Reality-Brille, einen Retina-Projektor, ein Head-Mounted-Display, eine Videobrille, ein Helmet-Mounted-Display und/oder dergleichen umfassen. Grundsätzlich können gängige Methoden und/oder Geräte als tragbare Anzeige verwendbar sind, die dazu geeignet sind, das Gesehene eines Benutzers virtuell zu erweitern. Mittels der tragbaren Anzeige kann also eine reale Umgebung, die von dem Benutzer gesehen wird, virtuell erweiterbar sein.

Mit "tragbar" kann grundsätzlich gemeint sein, dass die Anzeige durch die Körperkraft des Benutzers alleine haltbar und/oder bewegbar ist und direkt an dem Benutzer befestigt ist. Die Anzeige kann also mobil verwendbar und von dem Benutzer im getragenen Zustand ohne Weiteres bewegbar sein. Das kann bedeuten, dass die tragbare Anzeige leicht genug ist, dass der Benutzer diese über einen längeren Zeitraum bequem tragen kann und insbesondere keine unnötigen Einschränkungen für den Benutzer mit dem Tragen einhergehen. Der Benutzer kann die tragbare Anzeige insbesondere während des Durchführens einer Prozedur tragen. Dabei wirkt die tragbare Anzeige vorrangig unterstützend und schränkt den Benutzer insbesondere durch ihr Gewicht oder ihre räumliche Erstreckung nicht ein. In dem Zusammenhang kann sich der "getragene Zustand" auf einen Zustand beziehen, in dem die tragbare Anzeige am Kopf des Benutzers befestigt ist und derart angeordnet ist, dass Information visuell verfügbar machbar ist.

Das Sichtfeld kann sich auf einen Bereich beziehen, den der Benutzer gleichzeitig mit einem und/oder beiden Augen visuell erfassen kann, ohne den Kopf und/oder die Augen zu bewegen. Das kann bedeuten, dass das Sichtfeld das aktuell Gesehene des Benutzers umfasst. Insbesondere kann sich das Sichtfeld auf einen Bereich beziehen, den der Benutzer scharf sieht und/oder fokussiert visuell erfasst. Von diesem Bereich kann eine Peripherie zu unterscheiden sein, in dem der Benutzer lediglich unscharf sieht und/oder zumindest im Wesentlichen keine Details erkennen kann.

Unter einer "im Wesentlichen unbeeinträchtigten Betrachtung einer Umgebung in einem Sichtfeld des Benutzers" kann verstanden werden, dass der Benutzer die Umgebung ohne wesentliche Störungen oder Einschränkungen betrachten kann. Das kann insbesondere bedeuten, dass die Umgebung ohne störende Elemente und/oder Ablenkungen betrachtbar ist, wobei der Benutzer ein natürliches Sehverhalten anwenden kann. Die tragbare Anzeige kann also intuitiv anwendbar sein und schränkt den Benutzer in seinem Sichtfeld nicht ein. Vielmehr ist die tragbare Anzeige dazu eingerichtet, dem Sichtfeld weitere Information hinzuzufügen. Der Benutzer ist in seiner natürlichen Betrachtung der realen Welt nicht eingeschränkt.

Mittels der Vitalinformationseinblendung kann die Vitalinformation visuell für den Benutzer erfassbar gemacht werden. Die Vitalinformationseinblendung kann das von dem Benutzer Gesehene virtuell erweitern. Unter "visuell verfügbar machen" kann verstanden werden, dass der Benutzer einen quantitativen Wert des Vitalparameters aus der Vitalinformationseinblendung ablesen kann. Die Einblendung kann eine Darstellung umfassen. Beispielsweise kann die Einblendung die Darstellung einer Zahl umfassen, die einem quantitativen Wert eines Vitalparameters entspricht. Ferner kann die Vitalinformationseinblendung auch eine Darstellung eines pseudoquantitativen Verlaufs eines Vitalparameters umfassen. Unter einem Beruhen auf dem Vitalparameter kann auch eine Darstellung einer abstrahierten Information aus einem oder mehreren Vitalparametern, eines Verhältnisses verschiedener Vitalparameter und/oder dergleichen verstanden werden. Beispielsweise kann eine Darstellung eines sich in Abhängigkeit eines quantitativen Werts eines Vitalparameters in seiner Größe veränderndes Symbol eine auf dem Vitalparameter beruhende Vitalinformationseinblendung umfassen.

Die Vitalinformationseinblendung kann auf einem einzigen Vitalparameter beruhen und/oder auf mehreren unterschiedlichen Vitalparametern. Gemäß einigen Ausführungsformen können mehrere Vitalinformationseinblendungen vorgenommen werden, wobei jede der Vitalinformationseinblendungen zumindest teilweise auf zumindest einem unterschiedlichen Vitalparameter beruht. Die Vitalinformationseinblendungen können an derselben Stelle und/oder an unterschiedlichen Stellen im Sichtfeld des Benutzers einblendbar sein.

Die Vitalinformationseinblendung kann insbesondere derart in dem Sichtfeld des Benutzers einblendbar sein, dass sie im Wesentlichen nahtlos in die reale Umgebung integriert ist, um eine unbeeinträchtigte Betrachtung der Umgebung in dem Sichtfeld des Benutzers zu ermöglichen. Der Benutzer kann die Vitalinformationseinblendung bequem und/oder zumindest im Wesentlichen ohne Anstrengung wahrnehmen. Insbesondere kann das bedeuten, dass die Vitalinformationseinblendung derart einblendbar ist, dass sie in einem scharfen Sichtfeld des Benutzers für den Benutzer scharf visuell erfassbar ist. Es kann eine automatische Anpassung einer virtuellen Bildebene der Vitalinformationseinblendung vornehmbar sein, um zu erreichen, dass die Vitalinformationseinblendung für den Benutzers klar fokussiert ist. Dadurch können eine visuelle Ermüdung und/oder Unannehmlichkeiten vermieden und/oder reduziert werden. Der Benutzer kann die tragbare Anzeige intuitiv verwenden und die Vitalinformationseinblendung in gewisser Weise beiläufig wahrnehmen. Die Vitalinformationseinblendung kann derart einblendbar sein, dass der Benutzer in seinem Sichtfeld überwiegend und/oder vorrangig die Umgebung wahrnimmt. Die Vitalinformationseinblendung kann jedoch in dem scharfen Sichtfeld des Benutzers einblendbar sein, beziehungsweise in einem Bereich des Sichtfelds, in dem der Benutzer die Umgebung scharf erkennt. Beispielsweise kann die Vitalinformationseinblendung in einem Randbereich, beziehungsweise an den Seiten, des Sichtfelds, insbesondere des scharfen Sichtfelds, einblendbar sein. Der Benutzer kann dann vorrangig seinen Blick auf einen Patienten richten, etwa eine von ihm selbst vorgenommene Prozedur überwachen und mittels der Vitalinformationseinblendung im Randbereich des Sichtfelds die Vitalinformation erfassen, ohne den Blick vom Patienten abwenden zu müssen. Das kann bedeuten, dass der Benutzer die Vitalinformationseinblendung deutlich sehen kann, ohne seine Augen auf die Vitalinformationseinblendung zu akkommodieren.

Ferner verdeckt die Vitalinformationseinblendung auch keine wichtigen Details der Umgebung, die für die Vornahme der Prozedur wichtig sein könnten. Die Vitalinformationseinblendung kann derart an das Sichtfeld des Benutzers anpassbar sein, dass sie sich in das Sichtfeld einfügt und insbesondere ohne gesonderte Fokussierung auf die Vitalinformationseinblendung wahrnehmbar ist. Die Vitalinformationseinblendung kann an verschiedene Lichtverhältnisse anpassbar sein. Dadurch kann eine Anwendung mit zumindest im Wesentlichen gleichbleibender Qualität in unterschiedlichen Umgebungen realisierbar sein. Die Benutzerfreundlichkeit kann dadurch gewährleistet sein, dass die Vitalinformationseinblendung die natürliche Betrachtung der realen Umgebung durch den Benutzer nicht beeinträchtigt. Allgemein kann durch Art und Weise der Vornahme der Vitalinformationseinblendung ein immersives Benutzungserlebnis erreicht werden.

Mittels der Sichtfelderkennungseinheit kann das Sichtfeld des Benutzers erkannt werden und/oder ermittelt werden, welchen Teil der Umgebung der Benutzer visuell erfasst. Insbesondere kann dies eine Ermittlung der Blickrichtung des Benutzers und/oder eine Erfassung der Umgebung des Benutzers, insbesondere der Umgebung in Richtung der Blickrichtung, umfassen. Es kann erfasst werden, wohin der Benutzer blickt und/oder zumindest im Wesentlichen, was er sieht.

Mit einem Sichtfeldparameter kann ein quantitativer Wert gemeint sein, der sich zur Beschreibung des erkannten Sichtfelds eignet. Dadurch kann ein Sichtfeld beispielsweise in einem Koordinatensystem beschreibbar sein und/oder das Sichtfeld mit anderen Parametern vergleichbar sein. Ferner kann der Sichtfeldparameter etwa mittels einer elektronischen Datenverarbeitungseinrichtung verarbeitbar sein. Er kann folglich Information tragen, mittels der das Sichtfeld, insbesondere mathematisch und/oder geometrisch, beschreibbar ist. Beispielsweise kann mittels der Sichtfelderkennungseinheit eine Augenbewegung des Benutzers gemessen/ermittelt werden und/oder anhand der gemessenen Augenbewegung auf das Sichtfeld rückgeschlossen werden und/oder insbesondere eine relative Änderung des Sichtfelds ermittelt werden. Ferner kann mittels des Sichtfeldparameters ein Inhalt des Sichtfelds beschreibbar sein.

Die Sichtfelderkennungseinheit kann zumindest teilweise auf einer Eye-Tracking-Methode basieren und/oder eine Eye-Tracking-Methode implementieren. Die Sichtfelderkennungseinheit kann beispielsweise eine Infrarotlichtquelle umfassen, die dazu eingerichtet ist, die Augen des Benutzers mit Infrarotlicht zu bescheinen. Ferner kann die Sichtfelderkennungseinheit Kameras, insbesondere Infrarotkameras, und/oder Infrarotsensoren umfassen, die dazu eingerichtet sind, von den Augen reflektiertes Licht, insbesondere Infrarotlicht, räumlich zu erfassen. Mittels Bildverarbeitungsalgorithmen kann anhand des erfassten Lichts, insbesondere Infrarotlichts, die Blickrichtung des Benutzers verfolgt und/oder ermittelt werden. Ferner kann die Sichtfelderkennungseinheit eine Kamera umfassen, die dazu eingerichtet ist, die Umgebung des Benutzers, insbesondere zumindest im Wesentlichen in der Blickrichtung des Benutzers, abzubilden und Bilddaten zu erzeugen. Diese Bilddaten können Information über den Inhalt des Sichtfelds des Benutzers tragen. Andere Vorgehensweisen und/oder Einheiten können ebenfalls mit der Sichtfelderkennungseinheit gemeint sein. Die oben beschriebene Vorgehensweise ist lediglich beispielhaft zu verstehen.

Ein ermittelter Sichtfeldparameter kann einen Blickwinkel und/oder einen Blickkegel umfassen. Dieser kann räumlich, also in drei Raumrichtungen, aufgelöst sein. Mittels des Blickwinkels und/oder des Blickkegels kann das Sichtfeld beschreibbar sein. Ferner kann der Sichtfeldparameter eine Position zumindest eines Auges des Benutzers umfassen. Generell kann ein Sichtfeldparameter eine vektorielle Beschreibung des Sichtfelds und/oder der Blickrichtung in einem Bezugskoordinatensystem umfassen. Mittels des Bezugskoordinatensystems kann die Position des Benutzers im Raum beschreibbar sein. Das Bezugskoordinatensystem kann eine Referenz bieten, um die räumliche Lage und/oder Position des Benutzers und/oder der Umgebung zu definieren.

Ferner kann der Sichtfeldparameter Information darüber enthalten, welchen Teil der Umgebung der Benutzer scharf sieht. In diesem Zusammenhang kann mittels des Sichtfeldparameters eine Ausdehnung und/oder Reichweite des Sichtfelds des Benutzers beschreibbar sein, beziehungsweise kann der Sichtfeldparameter einen diesbezüglichen quantitativen Wert umfassen.

Außerdem kann der Sichtfeldparameter Information über die Umgebung des Benutzers umfassen. Beispielsweise kann der Sichtfeldparameter Bilddaten umfassen, die auf einer Abbildung der Umgebung beruhen. Beispielsweise können Bilderkennungsalgorithmen angewandt werden, um basierend auf dem Sichtfeldparameter Information über von dem Benutzer scharf gesehene Details zu ermitteln. Ergebnisse eines Bilderkennungsalgorithmus können ebenso mittels des Sichtfeldparameters übermittelbar sein. Es kann also grundsätzlich zumindest im Wesentlichen ermittelt werden, wohin der Benutzer geblickt hat und was er gesehen hat. In diesem Zusammenhang kann mit einem "Beziehen auf dem aktuellen Sichtfeld" gemeint sein, dass Information über das von dem Benutzer gemäß der Sichtfelderkennungseinheit Gesehene mittels des Sichtfeldparameters erfasst wird. Der Sichtfeldparameter kann Information über Details umfassen, die zwar in dem Sichtfeld des Benutzers angeordnet sind, von diesem jedoch nicht wahrnehmbar sind und/oder bewusst wahrgenommen werden. Beispielsweise kann der Sichtfeldparameter Information über den Inhalt einer Darstellung in dem Sichtfeld des Benutzers umfassen, wobei der Benutzer seinen Blick nicht vorrangig auf diese Darstellung richtet.

"In Abhängigkeit eines Parameters vornehmen oder unterlassen" kann bedeuten, dass eine Handlung und/oder Entscheidung auf dem Parameter, insbesondere einem Wert und/oder Informationsgehalt des Parameters, basieren kann. Der Parameter kann zumindest anteilhaft bestimmen, ob ein Vornehmen oder Unterlassen durchgeführt wird. In einigen Ausführungsformen kann das bloße Vorliegen eines Parameters ein Vornehmen oder Unterlassen bedingen. In anderen Ausführungsformen kann ein Wert des Parameters ein Vornehmen oder Unterlassen bedingen. Eine Entscheidung, ob ein Vornehmen oder Unterlassen durchzuführen ist, kann auf einer mathematischen Rechenvorschrift und/oder einem Algorithmus basieren, beispielsweise unter Anwendung künstlicher Intelligenz, maschinellen Lernens, Deep Learnings und/oder eines neuronalen Netzes, insbesondere eines offenen, geschlossen, einschichtigen und/oder rückgekoppelten neuronalen Netzes und/oder einer Kombination dieser.

"Basierend auf dem Sichtfeldparameter" kann in diesem Zusammenhang beispielsweise bedeuten, dass basierend auf dem Sichtfeld und/oder einem Inhalt des Sichtfelds des Benutzers die Einblendung der Vitalinformationseinblendung vorgenommen oder unterlassen wird. Das kann etwa bedeuten, dass, wenn der Benutzer seinen Kopf neigt, nach oben/unten blickt, zur Seite blickt und/oder dergleichen die Einblendung vorgenommen oder unterlassen wird. Denkbar ist, dass in einer Nullstellung, in der der Benutzer auf den Patienten blickt, die Einblendung vorgenommen wird. In einer von der Nullstellung abweichenden Stellung wird die Einblendung jedoch unterlassen. Ferner kann etwa erkannt werden, ob der Benutzer gerade beispielsweise keine Prozedur durchführt. In diesem Fall kann eine Einblendung unterlassen werden.

Hierin beschriebene Erkennungseinheiten können ebenso auf einer mathematischen Rechenvorschrift und/oder einem Algorithmus basieren, beispielsweise unter Anwendung künstlicher Intelligenz, maschinellen Lernens, Deep Learnings und/oder eines neuronalen Netzes, insbesondere eines offenen, geschlossen, einschichtigen und/oder rückgekoppelten neuronalen Netzes und/oder einer Kombination dieser.

Allgemein kann die Patientenüberwachungseinheit dazu eingerichtet sein, unterschiedliche Vitalparameter zu erfassen. Die tragbare Anzeige kann dann dazu eingerichtet sein, in dem getragenen Zustand mehrere unterschiedliche Vitalinformationseinblendungen in dem Sichtfeld des Benutzers einzublenden und/oder die unterschiedlichen Vitalparameter der Vitalinformationseinblendung zugrunde legen.

Dies kann insbesondere in Abhängigkeit des Sichtfeldparameters vorgenommen werden. Das kann bedeuten, dass eine der Vitalinformationseinblendungen vorgenommen und eine andere der Vitalinformationseinblendungen in Abhängigkeit von einem Parameter, insbesondere dem Sichtfeldparameter, vorgenommen oder unterlassen wird. Alternativ oder zusätzlich kann Information über einen anderen Vitalparameter mit derselben Vitalinformationseinblendung in Abhängigkeit von einem Parameter, insbesondere dem Sichtfeldparameter, visuell bereitgestellt werden.

Die Patientenzustandserkennungseinheit kann insbesondere dazu eingerichtet sein, anhand zumindest eines Vitalparameters den Zustand des Patienten zu ermitteln. Mit dem Zustand kann insbesondere der Vitalzustand gemeint sein und/oder generell eine aktuelle Verfassung des Patienten. Diese kann mittels der Vitalparameter definiert sein. Beispielsweise kann der Patient in einem ersten Vitalzustand eine Herzrate von 80 Schlägen pro Minute und in einem zweiten Vitalzustand 160 Schläge pro Minute aufweisen. Die Patientenzustandserkennungseinheit ist dazu eingerichtet, die Änderung dieses Vitalzustands zu erkennen. In einigen Ausführungsformen ist die Patientenzustandserkennungseinheit dazu eingerichtet, einen Verlauf zumindest einen Vitalparameters zu verfolgen und anhand des Verlaufs den Vitalzustand zu bestimmen. Ferner kann die Patientenzustandserkennungseinheit dazu eingerichtet sein, den zumindest einen Vitalparameter mit Grenzwerten zu vergleichen und anhand eines Vergleichsergebnisses den Vitalzustand zu bestimmen. Alternativ oder zusätzlich kann die Patientenzustandserkennungseinheit dazu eingerichtet sein, aus mehreren Vitalparametern den Vitalzustand zu ermitteln. Beispielsweise können die Vitalparameter verglichen und/oder in ein Verhältnis gesetzt werden. Es können Quotienten gebildet werden und/oder Vergleiche mit Referenzwerten durchgeführt werden. Insbesondere ist die Patientenzustandserkennungseinheit dazu eingerichtet, Anomalien des Vitalzustands zu erkennen. Allgemein kann mittels der Patientenzustandserkennungseinheit erkannt werden, ob eine Vitalinformationseinblendung, insbesondere beruhend auf einem bestimmen Vitalparameter, notwendig, hilfreich und/oder wichtig ist, oder ob die Vitalinformationseinblendung eher eine störende Wirkung auf den Benutzer haben könnte.

Der Vitalzustand kann sich grundsätzlich auf einen aktuellen physiologischen Zustand eines Patienten beziehen. Der Vitalzustand kann sich auf eine Gesamtheit an physiologischen Parametern und/oder Vitalparametern beziehen. In einigen Ausführungsformen kann sich der Vitalzustand auf die für ein Überleben des Patienten wichtigsten Vitalparameter beziehen. Diese können etwa die Herzfrequenz, den Blutdruck, die Atemfrequenz und die Körpertemperatur umfassen.

Mittels des Patientenzustandsparameters, der sich auf einen aktuellen Vitalzustand des Patienten bezieht, kann der Vitalzustand quantifiziert und/oder vergleichbar gemacht werden. In einigen Ausführungsformen kann der Patientenzustandsparameter einen Vitalparameter umfassen und/oder auf diesem beruhen. Der Patientenzustandsparameter kann einen quantitativen Wert umfassen, anhand dessen der Vitalzustand beurteilbar ist. Mittels des Patientenzustandsparameters kann in einigen Ausführungsformen auf einen Vitalparameter schließbar sein, der für den Benutzer von vorrangiger Bedeutung ist. Die Vitalinformationseinblendung kann auf diesem Vitalparameter beruhen.

Indem in Abhängigkeit des Patientenzustandsparameters die Vitalinformationseinblendung vorgenommen oder unterlassen wird, kann ein Benutzer auf den Vitalzustand des Patienten aufmerksam gemacht werden. Beispielsweise kann die Vitalinformationseinblendung vorgenommen werden, wenn anhand des Patientenzustandsparameter und/oder mittels der Patientenzustandserkennungseinheit eine Anomalie des Vitalzustands erkannt wurde. Ferner kann die Vitalinformationseinblendung vorgenommen werden, wenn eine Änderung des Vitalzustands erkannt wird, beispielsweise anhand des Patientenzustandsparameters.

Wie bereits beschrieben kann sich eine Prozedur beispielsweise auf eine therapeutische, diagnostische und/oder chirurgische Maßnahme beziehen. In anderen Worten kann die Prozedur etwa einen chirurgischen Eingriff, eine endoskopische Untersuchung, lebensrettende Maßnahmen in der Notfallmedizin und/oder dergleichen umfassen. Eine Prozedur kann einen komplexen Handlungsablauf umfassen, der in unterschiedliche, mehrere Schritte unterteilbar ist, die von dem Benutzer durchzuführen sind. Mittels des Prozedurschrittparameters kann die Prozedurschritterkennungseinheit einen quantitativen Wert bereitstellen, anhand dessen bestimmbar ist, in welchem Schritt von den mehreren Schritten der vom Benutzer am Patienten durchzuführenden Prozedur sich der Benutzer aktuell befindet. Es kann also ein Vergleichswert bereitgestellt werden, anhand dessen sich ein Fortschritt der Prozedur und/oder des Handlungsablaufs beurteilbar und/oder bestimmbar ist. Bezieht sich die Prozedur etwas auf eine mikroinvasive Laparoskopie kann ein erster Schritt ein Vornehmen einer Inzision, ein zweiter Schritt ein Bereitstellen eines Endoskops, ein dritter Schritt ein Einführen des Endoskops in den Bauchraum und ein vierter Schritt eine bildgebende Untersuchung des Bauchraums umfassen. Die Prozedurschritterkennungseinheit kann dazu eingerichtet sein, zu erkennen, in welchem dieser Schritte sich der Benutzer gerade befindet. Um mittels einer Recheneinheit einen Vergleichswert bereitzustellen, anhand dessen der aktuelle Schritt identifizierbar ist, kann die Prozedurschritterkennungseinheit dazu eingerichtet sein, den Prozedurschrittparameter bereitzustellen und/oder zu ermitteln.

Einer der Schritte kann eine Vitalinformationseinblendung beruhend auf einem Vitalparameter notwendiger machen als ein anderer der Schritte. In anderen Worten kann für den Benutzer bei einem der Schritte eine bestimmte Vitalinformation wichtiger sein als eine unterschiedliche Vitalinformation. Die tragbare Anzeige kann dies bei dem Vornehmen oder Unterlassen der Vitalinformationseinblendung berücksichtigen. Wird etwa die Inzision durchgeführt, kann es wichtig sein, Information über den Blutdruck einzublenden. Etwa eine Sauerstoffsättigung kann nachrangig wichtig sein. Wird hingegen die bildgebende Untersuchung durchgeführt, kann die Sauerstoffsättigung vorrangig wichtig sein. Eine Vitalinformationseinblendung kann dann auf der Sauerstoffsättigung beruhen und Information über den Blutdruck nicht eingeblendet sein.

Außerdem kann der Sichtfeldparameter Information bezüglich des Inhalts des Sichtfelds des Benutzers umfassen. Vorteilhafterweise kann dadurch das Vornehmen oder Unterlassen der Vitalinformationseinblendung auf dem Inhalt des Sichtfelds beruhen. Beispielsweise kann Information bezüglich des Inhalts des Sichtfelds Personen, Gegenstände und/oder dergleichen umfassen. Allgemein kann die Information bezüglich des Inhalts alles Gesehene des Benutzers umfassen.

Ferner kann das medizinische System eine Anzeigevorrichtung umfassen, die dazu eingerichtet ist, eine Vitalinformationsdarstellung anzuzeigen, die auf dem Vitalparameter beruht, wobei die tragbare Anzeige dazu eingerichtet ist, die Einblendung der Vitalinformationseinblendung zu unterlassen, wenn der Sichtfeldparameter anzeigt, dass sich die Anzeigevorrichtung in dem Sichtfeld des Benutzers befindet. Vorteilhafterweise kann dadurch verhindert werden, dass durch die Vitalinformationseinblendung in bestimmten Situationen eine unvorteilhafte Informationsbereitstellung vorgenommen wird. Es kann erreicht werden, dass der Benutzer nicht durch die Vitalinformationseinblendung abgelenkt wird. Ferner kann erreicht werden, dass Information nicht mehrfach bereitgestellt wird. Es kann etwa erkannt werden, dass der Benutzer gerade auf ein Pulsmessgerät blickt, das den Puls anzeigt. Eine Vitalinformationseinblendung beruhend auf dem Puls kann dann unterlassen werden.

Ferner kann das medizinische System ein medizinisches Bildgebungsinstrument umfassen, das dazu eingerichtet ist, Bilddaten zu erzeugen und die Bilddaten zu übertragen. Zudem kann das medizinische System eine Anzeigevorrichtung umfassen, die dazu eingerichtet ist, die Bilddaten zu empfangen und eine Darstellung für einen Benutzer zu erzeugen. Dabei kann die tragbare Anzeige dazu eingerichtet sein, die Einblendung der Vitalinformationseinblendung zu unterlassen, wenn der Sichtfeldparameter anzeigt, dass sich die Anzeigevorrichtung in dem Sichtfeld des Benutzers befindet. Vorteilhafterweise kann verhindert werden, dass der Benutzer durch die Vitalinformationseinblendung abgelenkt wird, wenn er auf die Anzeigevorrichtung blickt. Das medizinisches Bildgebungsinstrument kann beispielsweise ein Endoskop umfassen und der Benutzer eine endoskopische Untersuchung am Patienten durchführen. Dabei betrachtet der Benutzer die Darstellung auf der Anzeigevorrichtung, die auf den endoskopischen Bilddaten beruht. Eine Vitalinformationseinblendung könnte nachteilhaft sein, da sich der Benutzer vorrangig auf die endoskopische Untersuchung fokussieren sollte.

Zudem kann die tragbare Anzeige dazu eingerichtet sein, die Einblendung der Vitalinformationseinblendung vorzunehmen, wenn der Sichtfeldparameter anzeigt, das sich der Patient in dem Sichtfeld des Benutzers befindet. Vorteilhafterweise hat der Benutzer relevante Vitalinformation beim Blick auf den Patienten im Sichtfeld, um eine schnelle, effiziente und einfache Einschätzung des Vitalzustands des Patienten vornehmen zu können. Beispielsweise kann der Sichtfeldparameter solche Information bezüglich des Inhalts des Sichtfelds umfassen, dass der Benutzer gerade auf den Patienten blickt. In einem solchen Fall kann die Vitalinformationseinblendung vorgenommen werden. Wird jedoch erkannt, dass der Benutzer nicht auf den Patienten blickt und der Sichtfeldparameter entsprechend Information umfasst, dass der Patient nicht in dem Sichtfeld des Benutzers ist, kann die Vitalinformationseinblendung unterlassen werden. Denkbar ist etwa ein Einsatz der tragbaren Anzeige und/oder des medizinischen Systems während einer Visite eines Arztes in einem Krankenhaus. Dieser kann von einem Patienten zum nächsten gehen und bekommt entsprechende Vitalparameter beim Blick auf den neuen Patienten eingeblendet. Dadurch kann eine große Zeitersparnis erreicht werden.

Ein besonders mobiles und benutzerfreundliches medizinisches System kann bereitgestellt werden, wenn die Sichtfelderkennungseinheit an der tragbaren Anzeige angeordnet ist. Die tragbare Anzeige kann beispielsweise dazu eingerichtet sein, kabellos Information bezüglich des Vitalparameters und/oder der Vitalinformationseinblendung zu empfangen. Ein kompaktes System kann bereitgestellt werden.

Alternativ oder zusätzlich kann die Sichtfelderkennungseinheit separat von der tragbaren Anzeige angeordnet sein. Dadurch kann eine besonders leichte tragbare Anzeigevorrichtung bereitgestellt werden und/oder die Sichtfelderkennungseinheit präzisere und/oder akkuratere Sichtfeldparameter bestimmen. Beispielsweise kann die Sichtfelderkennungseinheit ein Kamerasystem umfassen, das in einem Operationssaal angeordnet ist. Ferner kann die Sichtfelderkennungseinheit an der Patientenüberwachungseinheit angeordnet sein. Dadurch kann schnell und einfach ermittelt werden, ob der Benutzer gerade auf die Patientenüberwachungseinheit blickt.

Gemäß einigen Ausführungsformen ist die Sichtfelderkennungseinheit dazu eingerichtet, die Blickrichtung des Benutzers zu ermitteln. Dabei kann der Sichtfeldparameter auf der ermittelten Blickrichtung beruhen. Dadurch kann die Entscheidung, ob die Vitalinformationseinblendung vorzunehmen oder zu unterlassen ist, auf der Blickrichtung des Benutzers beruhen. Insbesondere kann eine Änderung der Blickrichtung feststellbar sein und die Vitalinformationseinblendung auf der Änderung beruhen. Blickt der Benutzer etwa zu einer Seite, kann die Vitalinformationseinblendung vorgenommen oder unterlassen werden.

Grundsätzlich kann die tragbare Anzeige in eine optische Korrekturbrille integrierbar sein. Dadurch können Benutzer, die eine Sehschwäche haben, die tragbare Anzeige verwenden. Die tragbare Anzeige kann an etwa einen Aufsatz, insbesondere mittels eines Projektors und/oder Retinaprojektors, für die Korrekturbrille umfassen. Ferner ist auch eine integrale Ausbildung eines Korrekturglases mit einem Display denkbar.

Zudem kann die tragbare Anzeige eine Alarmvorrichtung umfassen, die dazu eingerichtet ist, den Benutzer zu alarmieren, wenn zumindest einer der Vitalparameter in einem kritischen Bereich ist. In kritischen Situationen kann die Aufmerksamkeit schnell und effizient erhöht werden und der Benutzer auf die kritische Situation hingewiesen werden. Insbesondere kann mittels der Patientenzustandserkennungseinheit ermittelbar sein, ob einer der Vitalparameter in einem kritischen Bereich ist.

Außerdem kann die tragbare Anzeige dazu eingerichtet sein, die Einblendung der Vitalinformationseinblendung unabhängig von dem Sichtfeldparameter vorzunehmen, wenn zumindest einer der Vitalparameter in einem kritischen Bereich ist. Es kann eine Überschreibungsfunktion (Override-Funktion) vorgesehen werden, die in kritischen Situationen zumindest im Wesentlichen zwangsläufig vorsieht, Information über kritische Vitalparameter bereitzustellen. In Notfällen kann erreicht werden, dass der Benutzer relevante Information schnell und einfach zur Verfügung hat. Insbesondere kann mittels der Patientenzustandserkennungseinheit ermittelbar sein, ob einer der Vitalparameter in einem kritischen Bereich ist.

Ferner kann die tragbare Anzeige dazu eingerichtet sein, die Einblendung der Vitalinformationseinblendung vorzunehmen, wenn der zumindest eine Patientenzustandsparameter in einem kritischen Bereich ist. Dabei kann die tragbare Anzeige dazu eingerichtet sein, die Einblendung der Vitalinformationseinblendung zu unterlassen, wenn der zumindest eine Patientenzustandsparameter nicht in dem kritischen Bereich ist. Die Vitalinformationseinblendung kann situationsspezifisch vorgenommen werden und insbesondere in Notfallsituationen vorgenommen werden. Der Benutzer wird nicht mit Information überladen, sondern bekommt insbesondere solche Information bereitgestellt, die vorrangig wichtig ist. Der kritische Bereich kann etwa einen Wertebereich eines Vitalparameters und/oder einer quantitativen Größe, die auf zumindest einem Vitalparameter beruht, oberhalb und/oder unterhalb eines Grenzwertes umfassen. Beispielsweise kann die Vitalinformationseinblendung beruhend auf der Herzfrequenz vorgenommen werden, wenn diese über 120 Schlägen pro Minuten ist. Der kritische Bereich kann zudem situationsspezifisch anpassbar sein. Bei manchen Prozeduren ist etwa eine Herzfrequenz von 120 Schlägen pro Minuten nicht kritisch, bei anderen Prozeduren hingegen schon. Bezüglich des Patientenzustandsparameters kann ein kritischer Bereich beispielsweise eine Berücksichtigung mehrerer Vitalparameter umfassen. Das kann bedeuten, dass nicht lediglich die Herzfrequenz mit einem Grenzwert verglichen wird. Es kann hingegen die Herzfrequenz, die Sauerstoffsättigung und die Atemfrequenz gemeinsam berücksichtigt werden, um festzustellen, ob der Patientenzustandsparameter in einem kritischen Bereich ist.

Zudem kann die tragbare Anzeige eine Alarmvorrichtung umfassen, die dazu eingerichtet ist, den Benutzer zu alarmieren, wenn der zumindest eine Patientenzustandsparameters in einem kritischen Bereich ist. In kritischen Situationen kann die Aufmerksamkeit schnell und effizient erhöht werden und der Benutzer auf die kritische Situation hingewiesen werden.

Der Benutzer kann besonders effizient und wirksam auf eine kritische Situation, einen kritischen Vitalparameter und/oder einen kritischen Patientenzustandsparameter aufmerksam gemacht werden, wenn die tragbare Anzeige dazu eingerichtet ist, den Benutzer mittels eines Vibrationsalarm zu alarmieren. Beispielsweise kann die tragbare Anzeige eine Brille, insbesondere eine AR-Brille und/oder eine VR-Brille umfassen, die Bügel aufweist. Eine Vorrichtung zur Erzeugung des Vibrationsalarms kann in den Bügeln angeordnet sein. Vorteilhaft kann grundsätzlich sein, wenn eine zur Bereitstellung des Vibrationsalarms eingerichtete Vibrationsvorrichtung in eine Befestigung der tragbaren Anzeige integriert ist, wobei die Befestigung zur Befestigung der tragbaren Anzeige am Kopf des Benutzers eingerichtet ist.

Alternativ oder zusätzlich kann die tragbare Anzeige dazu eingerichtet sein, den Benutzer mittels eines akustischen Alarmsignals zu alarmieren. Ein akustisches Warnsignal ist einfach bereitzustellen. In einigen Ausführungsformen ist die tragbare Anzeige dazu eingerichtet, den Benutzer mittels mehrerer verschiedenen Alarmsignale zu alarmieren. Beispielsweise kann ein akustisches Alarmsignal und ein Vibrationsalarm vorgesehen werden. Zumindest ein Alarmsignal kann derart bereitstellbar sein, dass es sich nach der Dringlichkeit der kritischen Situation richtet. Ist der Patient etwa in Lebensgefahr, kann das akustische Alarmsignal lauter sein.

Außerdem kann die tragbare Anzeige dazu eingerichtet sein, die Vitalinformationseinblendung in Abhängigkeit des Patientenzustandsparameters visuell zu verstärken. Der Benutzer wird dadurch besonders effizient etwa auf eine Änderung des Vitalzustands des Patienten aufmerksam gemacht. Eine derartige Methode der Erlangung der Aufmerksamkeit des Benutzers ist besonders verlässlich. Der Benutzer sieht sofort während der Prozedur eine Veränderung des Vitalzustands des Patienten. Insbesondere kann dadurch eine Reaktion des Patienten auf eine Prozedur schnell und einfach durch den Benutzer festgestellt werden. "Visuelles Verstärken" kann bedeuten, dass die Vitalinformationseinblendung farblich verändert wird, sich in der Helligkeit ändert, eine Transparenz reduziert wird, ein Kontrast erhöht wird, ein Hintergrund verändert wird und/oder dergleichen.

Alternativ oder zusätzlich kann die tragbare Anzeige dazu eingerichtet sein, die Vitalinformationseinblendung in Abhängigkeit des Patientenzustandsparameters zu vergrößern. Situationsspezifisch kann etwa eine Einblendung beruhend auf einen im Moment wichtigeren Vitalparameter größer dargestellt werden und dadurch erreicht werden, dass der Benutzer diese eher wahrnimmt.

Zudem kann die tragbare Anzeige dazu eingerichtet sein, die Vitalinformationseinblendung in Abhängigkeit des Patientenzustandsparameters zumindest teilweise blinkend vorzunehmen. Eine blinkende Darstellung wird durch den Benutzer besonders verlässlich wahrgenommen. Es kann sichergestellt werden, dass der Benutzer im Augenblick wichtige Vitalparameter wahrnimmt.

Außerdem kann die tragbare Anzeige dazu eingerichtet sein, die Vitalinformationseinblendung unabhängig von einer Blickrichtung des Benutzers vorzunehmen. Es kann dadurch sichergestellt werden, dass im Augenblick wichtige Vitalinformation in zumindest im Wesentlichen jedem Falle, beziehungsweise unabhängig von der Blickrichtung, eingeblendet wird.

Gemäß einigen Ausführungsformen kann die Patientenüberwachungseinheit dazu eingerichtet sein, unterschiedliche Vitalparameter zu erfassen, wobei die tragbare Anzeige dazu eingerichtet ist, in dem getragenen Zustand mehrere unterschiedliche Vitalinformationseinblendungen in dem Sichtfeld des Benutzers einzublenden, die auf unterschiedlichen Vitalparametern beruhen, und wobei die tragbare Anzeige dazu eingerichtet ist, in Abhängigkeit des jeweiligen von der Prozedurschritterkennungseinheit erkannten Schritts der durchzuführenden Prozedur jeweils zumindest eine der mehreren unterschiedlichen Vitalinformationseinblendungen vorzunehmen. Vorteilhafterweise wird Information über den Vitalparameter bereitgestellt, der für die Durchführung des aktuellen Schrittes von vorrangiger Bedeutung ist. Dem Benutzer wird zielgerichtet Information bereitgestellt und dieser dadurch effizient bei der Durchführung der Prozedur unterstützt. In diesem Zusammenhang kann unter einer Vitalinformationseinblendung eine Einblendung pro Vitalparameter verstanden werden. Beispielweise kann es eine Vitalinformationseinblendung für den Blutdruck und eine Vitalinformationseinblendung für den Puls geben.

Ferner kann die tragbare Anzeige dazu eingerichtet sein, die Vitalinformationseinblendung in Abhängigkeit des Prozedurschrittparameters visuell zu verstärken. Eine derartige Methode der Erlangung der Aufmerksamkeit des Benutzers ist besonders verlässlich. Bei einem ersten Schritt kann eine Vitalinformationseinblendung wichtiger sein als bei einem unterschiedlichen Schritt. In dem ersten Schritt kann die Vitalinformationseinblendung folglich visuell verstärkt werden. Der Benutzer sieht sofort während der Prozedur eine Veränderung des Vitalzustands des Patienten. Insbesondere kann dadurch eine Reaktion des Patienten auf eine Prozedur schnell und einfach durch den Benutzer festgestellt werden. "Visuelles Verstärken" kann bedeuten, dass die Vitalinformationseinblendung farblich verändert wird, sich in der Helligkeit ändert, eine Transparenz reduziert wird und/oder dergleichen.

Außerdem kann die tragbare Anzeige dazu eingerichtet sein, die Vitalinformationseinblendung in Abhängigkeit des Prozedurschrittparameters zu vergrößern. Schrittspezifisch kann etwa eine Vitalinformationseinblendung beruhend auf einen im Moment wichtigeren Vitalparameter größer dargestellt werden und dadurch erreicht werden, dass der Benutzer diese eher wahrnimmt.

Außerdem kann die tragbare Anzeige dazu eingerichtet sein, die Vitalinformationseinblendung in Abhängigkeit des Prozedurschrittparameters zumindest teilweise blinkend vorzunehmen. Eine blinkende Darstellung wird durch den Benutzer besonders verlässlich wahrgenommen. Es kann sichergestellt werden, dass der Benutzer im Augenblick wichtige Vitalparameter wahrnimmt.

Zudem kann die tragbare Anzeige dazu eingerichtet sein, die Vitalinformationseinblendung unabhängig von einer Blickrichtung des Benutzers vorzunehmen. Es kann dadurch sichergestellt werden, dass im Augenblick wichtige Vitalinformation in zumindest im Wesentlichen jedem Falle, beziehungsweise unabhängig von der Blickrichtung, eingeblendet wird.

Außerdem kann die Prozedurschritterkennungseinheit eine Kamera umfassen, die dazu eingerichtet ist, Bilddaten zu erzeugen, und die Prozedurschritterkennungseinheit dazu eingerichtet sein, den zumindest einen Prozedurschrittparameter anhand der Bilddaten zu ermitteln. Dadurch wird eine sichere und effiziente Schritterkennung erreicht. Die Prozedurschritterkennungseinheit kann eine Bilderkennungseinheit umfassen. Diese kann auf maschinellem Lernen basieren und/oder ein neuronales Netz umfassen.

Zudem kann die Kamera an der tragbaren Anzeige angeordnet sein. Dadurch kann ein kompaktes und/oder mobil einsetzbares medizinisches System bereitgestellt werden. Die Kamera kann ähnlich einer Kamera für die Sichtfelderkennung angeordnet sein. Es kann eine gemeinsame Kamera für die Sichtfelderkennung und Prozedurschritterkennung vorgesehen sein. Allgemein kann die tragbare Anzeige eine Kamera umfassen, die dazu eingerichtet ist, Bilddaten der Umgebung des Benutzers, insbesondere in der Blickrichtung des Benutzers, zu erzeugen. Ferner kann die Prozedurschritterkennungseinheit zumindest teilweise an der tragbaren Anzeige angeordnet sein.

Gemäß einigen Ausführungsformen kann die Kamera separat von der tragbaren Anzeige angeordnet sein. Es kann etwa dadurch unabhängig von der Blickrichtung des Benutzers und/oder der Anwesenheit des Benutzers der Prozedurschrittparameter ermittelbar sein. Der Patient kann besonders sicher überwacht werden.

Gemäß einigen Ausführungsformen kann die Prozedur eine Intubation des Patienten sein. Vorteilhafterweise kann der Benutzer in jedem Schritt der Intubation über in dem entsprechenden Schritt relevante Vitalinformation unterrichtet werden. Besonders relevant kann dies bei einer Einschätzung sein, ob ein Tubus, der der Luftröhre zugeführt werden sollte, tatsächlich dieser zugeführt wurde. Es kann vorkommen, dass der Tubus fälschlicherweise der Speiseröhre zugeführt wird. In einem solchen Fall würde beispielsweise die Sauerstoffsättigung abfallen und Auffälligkeiten in einer Atemgasanalyse entstehen. Der Benutzer kann etwa während des Einschaltens der Beatmungsmaschine darüber in seinem Sichtfeld informiert werden, ob der Tubus richtig gelegt wurde. Dabei muss der Benutzer seinen Blick nicht von dem Patienten ablassen, sondern kann den Patienten beobachten, während die Beatmung beginnt, und gleichzeitig Information über die entsprechenden Vitalparameter bekommen.

Das medizinische System kann ferner eine Patientenzustandserkennungseinheit, die dazu eingerichtet ist, zumindest einen Patientenzustandsparameter zu ermitteln, der sich auf einen aktuellen Vitalzustand des Patienten bezieht, umfassen. Es können vorteilhafterweise mehrere hierin beschriebene Aspekte kombiniert werden und ein besonders leistungsstarkes medizinisches System bereitgestellt werden.

In dem Zusammenhang kann die tragbare Anzeige dazu eingerichtet sein, eine Einblendung der Vitalinformationseinblendung in Abhängigkeit des Prozedurschrittparameters und des Patientenzustandsparameter vorzunehmen oder zu unterlassen. Eine Sicherheit des Patienten kann verbessert werden, da zum einen auf den aktuellen Schritt Rücksicht genommen wird, zum anderen jedoch auch überprüft wird, ob sich der Vitalzustand verschlechtert. Der Benutzer kann durch entsprechende Vitalinformationseinblendungen zielgerichtet und effizient unterrichtet und/oder unterstützt werden.

Außerdem kann die tragbare Anzeige dazu eingerichtet sein, dann, wenn der Patientenzustandsparameter einen kritischen Vitalzustand des Patienten anzeigt, eine Einblendung der Vitalinformationseinblendung unabhängig von dem Prozedurschrittparameter vorzunehmen oder zu unterlassen. Beispielsweise in Notfallsituationen kann sichergestellt werden, dass der Benutzer Information über alle notwendigen Vitalparameter erhält.

Im Folgenden wird die vorliegende Erfindung anhand der beigefügten Figuren beispielhaft beschrieben. Die Zeichnung, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: eine schematische Darstellung eines medizinischen Systems;
- Fig. 2: eine weitere schematische Darstellung des medizinischen Systems;
- Fig. 3: eine schematische Darstellung eines Sichtfelds eines Benutzers;
- Fig. 4: eine weitere schematische Darstellung eines Sichtfelds eines Benutzers;
- Fig. 5: eine weitere schematische Darstellung eines Sichtfelds eines Benutzers;
- Fig. 6: eine weitere schematische Darstellung des medizinischen Systems;
- Fig. 7: eine weitere schematische Darstellung eines Sichtfelds eines Benutzers;
- Fig. 8: eine weitere schematische Darstellung eines Sichtfelds eines Benutzers;
- Fig. 9: eine schematische Darstellung einer tragbaren Anzeige; und
- Fig. 10: ein schematisches Ablaufdiagramm eines Verfahrens.

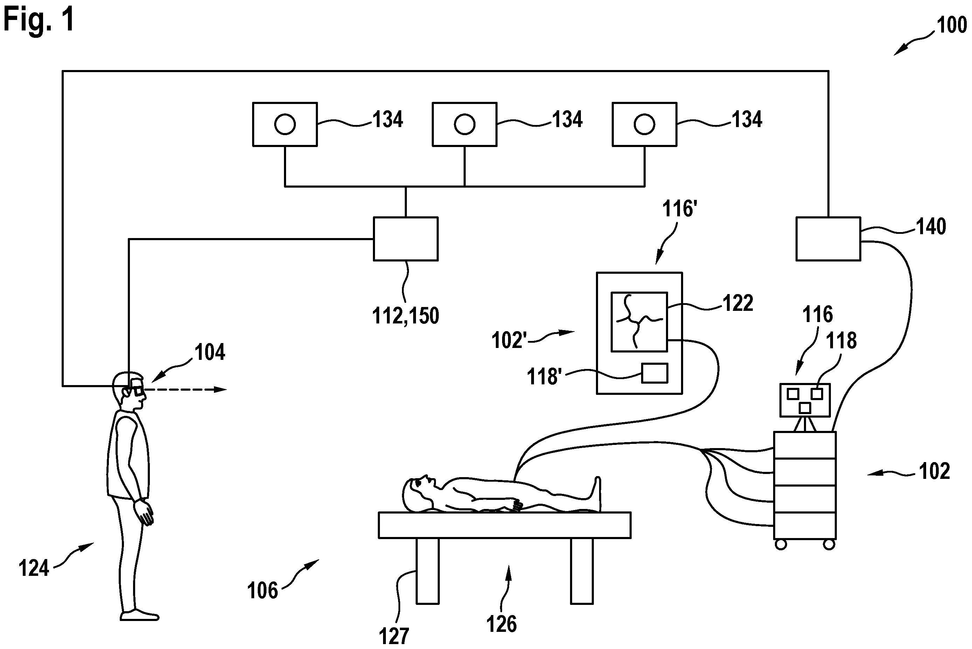

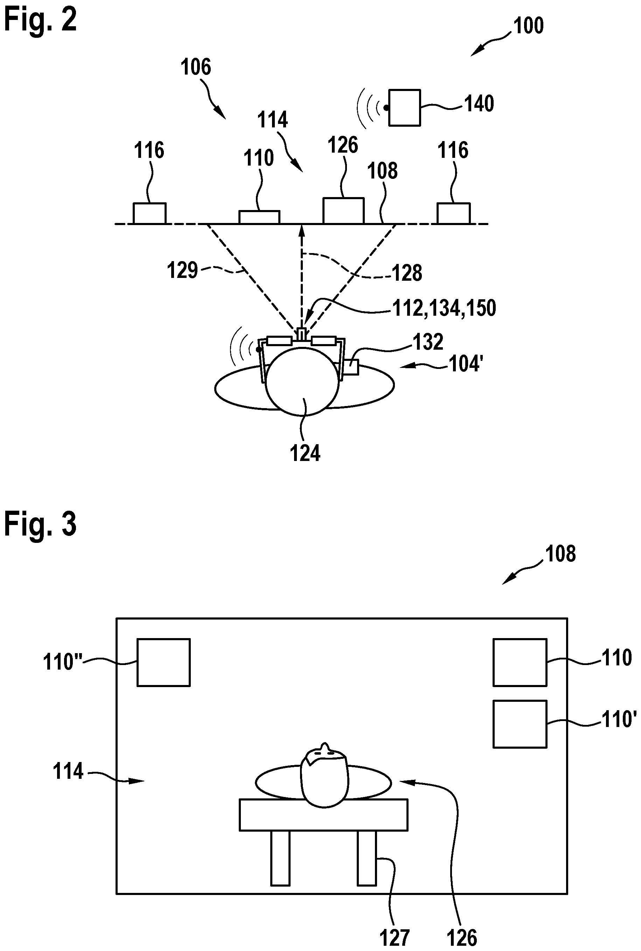

Fig. 1 zeigt eine schematische Darstellung eines medizinischen Systems 100. Man erkennt einen Benutzer 124, der eine tragbare Anzeige 104 am Kopf trägt. Die tragbare Anzeige 104 umfasst gemäß Fig. 1 eine VR-Brille und ist dazu eingerichtet, eine Einblendung in einem Sichtfeld 108 (siehe z.B. Fig. 2) des Benutzers 124 vorzunehmen und gestattet dabei eine zumindest im Wesentlichen unbeeinträchtigte Betrachtung einer Umgebung 106 in dem Sichtfeld 108 des Benutzers 124. Die Einblendung kann die von dem Benutzer 124 gesehen reale Umgebung 106 virtuell erweitern und umfasst insbesondere Information über einen Vitalzustand eines Patienten 126. Die tragbare Anzeige 104 ist dazu eingerichtet, in dem getragenen Zustand eine Vitalinformationseinblendung 110 in dem Sichtfeld 108 des Benutzers 124 einzublenden, die auf einem Vitalparameter beruht.

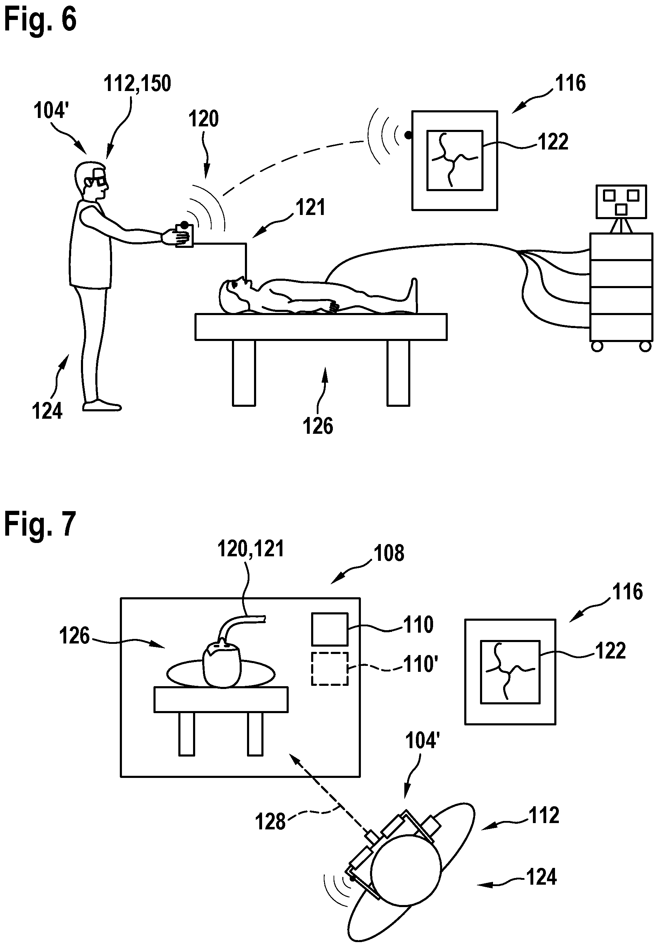

In der Fig. 1 ist beispielhaft das medizinische System 100 in einer stationären Anwendung in einem Operationssaal gezeigt. Ebenso denkbar ist eine mobile Anwendung in einem Rettungswagen oder dergleichen. Der Patient 126 liegt auf einer Behandlungsliege 127 und der Benutzer 124 nimmt eine Prozedur an dem Patienten 126 vor. Die Prozedur kann ein operativer Eingriff, eine diagnostische Handlung und/oder eine Überwachung eines Vitalzustands des Patienten umfassen. In der Fig. 6 ist eine schematische Darstellung des medizinischen Systems 100 gezeigt, wobei die Prozedur eine Intubation des Patienten 126 mittels eines Videostiletts ist.

Der Vitalzustand des Patienten 126 wird mittels einer Patientenüberwachungseinheit 102 überwacht. Diese ist dazu eingerichtet, mehrere unterschiedliche Vitalparameter des Patienten 126 zu erfassen. Die Patientenüberwachungseinheit 102 umfasst dazu mehrere Module, die jeweils zumindest einen Vitalparameter erfassen. Beispielsweise ist ein Modul ein Thermometer und erfasst die Temperatur des Patienten. Ein anderes Modul ist ein Pulsoxymeter und erfasst die Sauerstoffsättigung im Blut. Ferner umfasst die Patientenüberwachungseinheit 102 eine Anzeigevorrichtung 116, die dazu eingerichtet ist, eine Vitalinformationsdarstellung 118 anzuzeigen, die auf dem Vitalparameter beruht. Beispielsweise wird ein Verlauf des Blutdrucks angezeigt, ein Puls des Patienten, eine Atemfrequenz und/oder dergleichen. Die Anzeigevorrichtung 116 ist eine zentrale Anzeigevorrichtung, die dazu eingerichtet ist, mehrere Vitalinformationsdarstellungen 118 anzuzeigen, die auf unterschiedlichen Vitalparametern beruhen.

Zusätzlich ist zumindest eine zweite Patientenüberwachungseinheit 102` vorgesehen. Zur Erhöhung der Übersichtlichkeit werden die Bezugszeichen ähnlicher, verwandter und/oder mehrfach vorkommender Einheiten mit Hochkommata gekennzeichnet. Die zweite Patientenüberwachungseinheit 102` umfasst ebenfalls eine Anzeigevorrichtung 116`, an der eine Vitalinformationsdarstellungen 118' angezeigt wird. Zusätzlich wird an der Anzeigevorrichtung 116` eine Darstellung 122 für den Benutzer 124 erzeugt, die auf Bilddaten eines medizinischen Bildgebungsinstruments 120 (siehe Fig. 6), insbesondere Endoskops, beruht. Die Anzeigevorrichtung 116' kann beispielsweise einen mobilen Monitor eines Endoskops umfassen.

Die Information über den Vitalzustand des Patienten 126 und/oder Darstellungen beruhend auf einem Vitalparameter können dem Benutzer, wie bereits beschrieben, in dem Sichtfeld eingeblendet werden. Das Vornehmen der Einblendung kann bedarfsweise, situationsabhängig und/oder zielgerichtet vorgenommen oder unterlassen werden, sodass dem Benutzer 124 lediglich solche Information über sein Sichtfeld 108 bereitgestellt wird, die vorrangig wichtig ist. Dies wird im Folgenden genauer erklärt.

Um zu ermitteln, welche Information im Augenblick vorrangig wichtig für den Benutzer 124 ist, umfasst das medizinische System 100 eine Sichtfelderkennungseinheit 112, eine Patientenzustandserkennungseinheit 140 und eine Prozedurschritterkennungseinheit 150. In anderen Ausführungsformen kann auch nur eine oder zwei der Einheiten vorgesehen sein. Die Einheiten 112, 140, 150 können zum Zusammenwirken vorgesehen sein.

Die Sichtfelderkennungseinheit 112 ist dazu eingerichtet, zumindest einen Sichtfeldparameter zu ermitteln, der sich auf das aktuelles Sichtfeld 108 des Benutzers 124 bezieht. Anhand des Sichtfeldparameters kann die tragbare Anzeige 104 entscheiden, ob eine Einblendung der Vitalinformationseinblendung 110 in Abhängigkeit des Sichtfeldparameters vorzunehmen oder zu unterlassen ist. Anhand der folgenden Figuren werden einige Beispiele dafür erklärt, auf welcher Grundlage die Entscheidung getroffen werden könnte und/oder welche Information der Sichtfeldparameter tragen könnte. Allgemein könnte die Einblendung unterlassen werden, wenn der Benutzer 124 auf eine der Anzeigevorrichtungen 116, 116` blickt und auf dieser ohnehin eine Darstellung 118 beruhend auf dem Vitalparameter angezeigt wird, auf dem die Vitalinformationseinblendung 110 der tragbaren Anzeige 104 beruhen würde. In anderen Worten kann eine Einblendung eines Pulses des Patienten 126 unterlassen werden, wenn ohnehin eine Darstellung beruhend auf dem Puls des Patienten 126 in dem Sichtfeld 108 des Benutzers 124 ist. Es wird also nicht mehrfach dieselbe Information bereitgestellt. Der Benutzer wird dadurch weniger abgelenkt.

Andererseits kann ebenso erkannt werden, dass keine Darstellung beruhend auf dem Puls in dem Sichtfeld 108 ist. Eine Einblendung einer entsprechenden Vitalinformationseinblendung kann dann vorgenommen werden. Dadurch erlangt der Benutzer 124 schnell und einfach Information über den Puls, ohne auf eine eine entsprechende Darstellung anzeigende Anzeigevorrichtung blicken zu müssen. Beispielsweise kann er sich dadurch besser auf eine Operation konzentrieren, die er gerade am Patienten 126 vornimmt. Grundsätzlich denkbar ist, dass die Vitalinformationseinblendung abhängig von einer Blickrichtung 128 des Benutzers 124 und/oder einem Inhalt 114 des Sichtfelds 108 ist. Der Inhalt 114 kann etwa den Patienten 124 und/oder eine der Anzeigevorrichtungen 116, 116' umfassen.

Die Sichtfelderkennungseinheit 112 kann an der tragbaren Anzeige 104 und/oder separat von der tragbaren Anzeige 104 angeordnet sein. In der Fig. 1 sind drei Kameras 134 gezeigt, die dazu eingerichtet sind, Bilddaten von der Umgebung 106, des Benutzers 124 und/oder des Patienten 126 zu erfassen. Dadurch kann räumliche Information betreffend den Benutzer und/oder sein Verhalten gewonnen werden. Die Kameras 134 können mit der Sichtfelderkennungseinheit 112 verbunden sein, beziehungsweise ein Teil dieser sein. Die Sichtfelderkennungseinheit 112 kann mittels der räumlichen Auflösung die Blickrichtung 128 des Benutzers 124 ermitteln und einen Sichtfeldparameter bereitstellen, der auf der ermittelten Blickrichtung 128 beruht. Generell kann mittels der Kameras 134 das von dem Benutzer 124 Gesehene ermittelt werden und auf Grundlage dessen die Einblendung der Vitalinformationseinblendung vorgenommen oder unterlassen werden.

Die Kameras 134 können zudem mit der Prozedurschritterkennungseinheit 150 verbunden sein, beziehungsweise Teil dieser sein. Die Prozedurschritterkennungseinheit 150 und die Sichtfelderkennungseinheit 112 können gemeinsam auf einer Recheneinheit implementiert sein. Die Prozedurschritterkennungseinheit 150 ist dazu eingerichtet, zumindest einen Prozedurschrittparameter zu ermitteln, der beschreibt, in welchem Schritt von mehreren Schritten einer vom Benutzer 124 am Patienten 126 durchzuführenden Prozedur sich der Benutzer 124 aktuell befindet. Dabei ist die tragbare Anzeige 104 dazu eingerichtet, eine Einblendung der Vitalinformationseinblendung 110 in Abhängigkeit des Prozedurschrittparameters vorzunehmen und/oder zu unterlassen. Wie bereits beschrieben, ist die Patientenüberwachungseinheit 102 dazu eingerichtet ist, unterschiedliche Vitalparameter zu erfassen. Basierend auf den unterschiedlichen Vitalparameter kann die tragbare Anzeige 104 ein Einblenden unterschiedlicher Vitalinformationseinblendungen 110 vornehmen oder unterlassen. Welche der Vitalinformationseinblendungen 110 vorgenommen wird, wird anhand des erkannten Schritts der durchzuführenden Prozedur erkannt.

Die Prozedurschritterkennungseinheit 150 ist dazu eingerichtet, den zumindest einen Prozedurschrittparameter anhand der Bilddaten zu ermitteln, die mittels der Kameras 134 erzeugt wurden. Mittels der Kameras 134 wird also die Prozedur überwacht und/oder analysiert. Anhand des Analyseergebnisses kann zielgerichtet die Einblendung der Vitalinformationseinblendung 110 vorgenommen oder unterlassen werden.

Mittels der Kameras 134 kann ebenso ein Patientenzustandsparameter ermittelt werden. Dieser wird grundsätzlich mittels der Patientenzustandserkennungseinheit 140 ermittelt, die dazu eingerichtet sein kann, Bilddaten der Kameras 134 zu analysieren. Der Patientenzustandsparameter bezieht sich auf einen Vitalzustand des Patienten. Er trägt Information über die physiologischen Zustandswerte des Patienten 126. Die tragbare Anzeige 104 ist dazu eingerichtet, eine Einblendung der Vitalinformationseinblendung 110 in Abhängigkeit des Patientenzustandsparameters vorzunehmen oder zu unterlassen. Ist der Vitalzustand des Patienten und/oder der Patientenzustandsparameter in einem kritischen Bereich, etwa weil die Sauerstoffsättigung stark abgefallen ist oder der Blutdruck stark angestiegen ist, kann die Einblendung vorgenommen werden. Bewegt sich die Sauerstoffsättigung oder der Blutdruck jedoch in einem normalen physiologischen Bereich, kann von einer Einblendung abgesehen werden. Vorrangig ist die Patientenzustandserkennungseinheit 140 jedoch dazu eingerichtet, Vitalparameter der Patientenüberwachungseinheit 102 zu analysieren, vergleichen und/oder dergleichen, um den Patientenzustandsparameter zu ermitteln und insbesondere, um festzustellen, ob der Patientenzustandsparameter in einem kritischen Bereich ist, beziehungsweise, ob der Vitalzustand des Patienten in einem kritischen Bereich ist.

Die Entscheidung, ob eine Einblendung vorgenommen oder unterlassen wird, kann auf Grundlage aller Parameter, also des Patientenzustandsparameters, des Sichtfeldparameters und des Prozedurschrittparameters vorgenommen werden. Etwa mittels eines Algorithmus kann eine Abwägung durchgeführt werden, ob die Einblendung vorgenommen wird oder nicht. Ferner weist das medizinische System eine Überschreibungsfunktion auf, die bedingt, dass zwangsläufig eine Einblendung einer Vitalinformationseinblendung 110 vorgenommen wird, wenn ein entsprechender Vitalparameter und/oder anderer Parameter in einem kritischen Bereich ist. Dies kann etwa unabhängig von dem Sichtfeldparameter und/oder dem Prozedurschrittparameter vorgenommen werden. Um den Benutzer zu alarmieren, ist eine Alarmvorrichtung 132 (siehe Fig. 2) vorgesehen. Das Alarmieren kann mittels eines Vibrationsalarms und/oder eines akustischen Alarmsignals durchgeführt werden.

Wie in den Fig. 3 bis 5 näher gezeigt, kann die Aufmerksamkeit des Benutzers 124 verstärkt auf die Vitalinformationseinblendung 110 gelenkt werden, wenn diese blinkend vorgenommen wird, sie optisch hervorgehoben wird, vergrößert wird. Dies kann in Abhängigkeit zumindest eines hierin beschriebenen Parameters durchgeführt werden.

In der Fig. 2 ist das medizinische System 100 in einer weiteren schematischen Darstellung gezeigt. Der Kopf des Benutzer 124 ist schematisch in einer Ansicht von oben dargestellt. Der Benutzer 124 trägt an seinem Kopf eine tragbare Anzeige 104`, die der Anzeige 104 sehr ähnlich ist. Anstelle der Anzeige 104 kann ebenso die Anzeige 104' und/oder eine andere erfindungsgemäße tragbare Anzeige verwendet werden. Die tragbare Anzeige 104` ist als VR-Brille vorgesehen.

Die tragbare Anzeige 104` umfasst die Sichtfelderkennungseinheit 112 und die Prozedurschritterkennungseinheit 150. Die Sichtfelderkennungseinheit 112 ist dazu eingerichtet, eine Blickrichtung 128 des Benutzers 124 zu ermitteln. Dazu umfasst die Sichtfelderkennungseinheit 112 umfasst eine Infrarotlichtquelle (nicht im Detail dargestellt), die dazu eingerichtet ist, die Augen des Benutzers 124 mit Infrarotlicht zu bescheinen und zumindest einen Infrarotsensor (nicht im Detail dargestellt), der dazu eingerichtet ist, von den Augen reflektiertes Infrarotlicht räumlich zu erfassen. Mittels eines Bildverarbeitungsalgorithmus wird anhand des erfassten Lichts Infrarotlichts die Blickrichtung 128 des Benutzers 124 verfolgt und/oder ermittelt. Zusätzlich kann dadurch und/oder durch weitere Sichtfelderkennungsmethoden das Sichtfeld 108 des Benutzers 124 ermittelt und/oder abgeschätzt werden. Insbesondere kann dies mittels einer Ermittlung eine Blickkegels 129 vorgenommen werden.

Ferner umfasst die Sichtfelderkennungseinheit 112 eine Kamera 134, die dazu eingerichtet ist, die Umgebung 106 des Benutzers 124 abzubilden und Bilddaten zu erzeugen. Die Kamera 134 ist derart ausgerichtet, dass ihre optische Achse zumindest im Wesentlichen parallel zu der Blickrichtung 128 angeordnet ist und/oder zumindest im Wesentlichen entlang der Blickrichtung 128 verläuft. Durch die Kamera 134 kann gemeinsam mit der Blickrichtung 128 das Sichtfeld 108 des Benutzers 124 abgeschätzt werden. Ferner ist die Sichtfelderkennungseinheit 112 dazu eingerichtet, zu ermitteln, worauf der Benutzer 124 seinen Blick fokussiert. Dadurch kann der Inhalt des Sichtfelds 108 des Benutzers ermittelt werden. Zudem kann ermittelt werden, was genau der Benutzer 124 gerade sieht.

Die mittels der Kamera 134, die an der tragbaren Anzeige 104' angeordnet ist, erzeugten Bilddaten können also Information über den Inhalt des Sichtfelds des Benutzers tragen. Diese Bilddaten können ebenfalls von der Prozedurschritterkennungseinheit 150 verwendet werden, um den Schritt der Prozedur zu erkennen. Die Kamera 134 kann als Teil der Prozedurschritterkennungseinheit 150 und der Sichtfelderkennungseinheit 112 angesehen werden. Die Kameras 134 gemäß der Fig. 1 können zusätzlich vorgesehen werden. Die tragbare Anzeige 104` eignet sich jedoch insbesondere auch für mobile Anwendungen.

Mittels der Bilddaten der Kamera 134 kann der Inhalt 114 in dem Sichtfeld 108 des Benutzers 124 ermittelt werden. So kann festgestellt werden, dass sich der Patient 126 in dem Sichtfeld 108 befindet. Ein entsprechender Sichtfeldparameter wird erstellt und auf Grundlage dieses Sichtfeldparameters entscheidet die tragbare Anzeige 104`, ob die Einblendung der Vitalinformationseinblendung 110 vorgenommen oder unterlassen wird. Gemäß der Fig. 2 soll die Vitalinformationseinblendung 110 vorgenommen werden, wenn der Patient 126 in dem Sichtfeld 108 des Benutzers 124 ist. Folglich wird die Einblendung der Vitalinformationseinblendung 110 vorgenommen. Diese wird derart in dem Sichtfeld 108 des Benutzers 124 eingeblendet, dass dieser die Vitalinformationseinblendung 110 scharf sieht, wenn er auf den Patienten 126 blickt. In der Fig. 2 ist dies dadurch schematisch dargestellt, dass sich die Vitalinformationseinblendung 110 und der Patient 126 zumindest teilweise in derselben Ebene befinden.

Man erkennt in der Fig. 2 zudem, dass in der Umgebung 106 des Benutzers 124 Anzeigevorrichtungen 116 angeordnet sind. Mittels der Sichtfelderkennungseinheit 112 wird jedoch erkannt, dass der Benutzer nicht auf die Anzeigevorrichtungen 116 blickt, beziehungsweise diese außerhalb des Sichtfelds 108 angeordnet sind. Die Kamera 134 kann jedoch dazu eingerichtet sein, die Umgebung 106 außerhalb des Sichtfelds 108 zumindest teilweise abzubilden. Es kann also erkannt werden, in welchem Winkel relativ zur Blickrichtung 128 eine Anzeigevorrichtung 116 angeordnet ist. Es kann dann beispielsweise blickrichtungsabhängig der Sichtfeldparameter bestimmt werden.

Ferner erkennt man in der Fig. 2, dass die tragbare Anzeige 104` kabellos mit der Patientenzustandserkennungseinheit 140 verbunden ist. So kann die tragbare Anzeige 104` die bereits beschriebenen Funktionen der Patientenzustandserkennungseinheit 140 implementieren. Insbesondere kann die Überschreibungsfunktion implementiert sein, die bewirkt, dass in einer Notfallsituation bestimmte Vitalinformationseinblendungen vorgenommen werden. Zur Erzeugung eines Alarmsignals umfasst die tragbare Anzeige 104` eine Alarmvorrichtung 132. Diese umfasst ein Mikrofon zur Erzeugung eines akustischen Alarmsignals und eine Vibrationsvorrichtung zur Erzeugung eines Vibrationsalarms. Die Vibrationsvorrichtung ist ein einem Bügel der tragbaren Anzeige 104' angeordnet.

In der Fig. 3 ist eine schematische Darstellung des Sichtfelds 108 des Benutzers 124 gezeigt, der während einer Prozedur auf den Patienten 126 blickt. Es wird schematisch das von dem Benutzer 124 Gesehene dargestellt. Der Patient 126 liegt auf der Behandlungsliege 127, wie es auch in der Fig. 1 gezeigt wird. Die Sichtfelderkennungseinheit 112 erkennt den Inhalt 114 des Sichtfelds 108 und erkennt folglich, dass der Patient 126 im Sichtfeld 108 des Benutzers 124 ist. Zudem wird auch erkannt, dass keine Anzeigevorrichtung im Sichtfeld 116 ist, auf der eine Vitalinformationsdarstellung angezeigt wird. Daher wird eine Einblendung von Vitalinformationseinblendungen 110, 110`, 110" vorgenommen. Diese erweitern virtuell die reale Umgebung des Benutzers 124 und werden von dem Benutzer am Rand des Sichtfelds 108 gesehen. Die Vitalinformationseinblendungen 110, 110`, 110" stören seine Sicht auf den Patienten 126 nicht. Beispielhaft sind drei Vitalinformationseinblendungen 110, 110`, 110" gezeigt. Es kann jedoch auch nur eine Vitalinformationseinblendung vorgenommen werden, diese kann Information über mehrere Vitalparameter übermitteln. Gemäß Fig. 3 wird etwa je ein Zahlenwert eingeblendet, der den Puls, die Herzfrequenz und die Sauerstoffsättigung wiedergibt. Die Darstellung gemäß Fig. 3 kann etwa dem Gesehenen des Benutzers gemäß der Fig. 2 entsprechen. Die Sichtfelderkennungseinheit 112 erkennt also, dass der Patient 126 im Sichtfeld 108 ist.

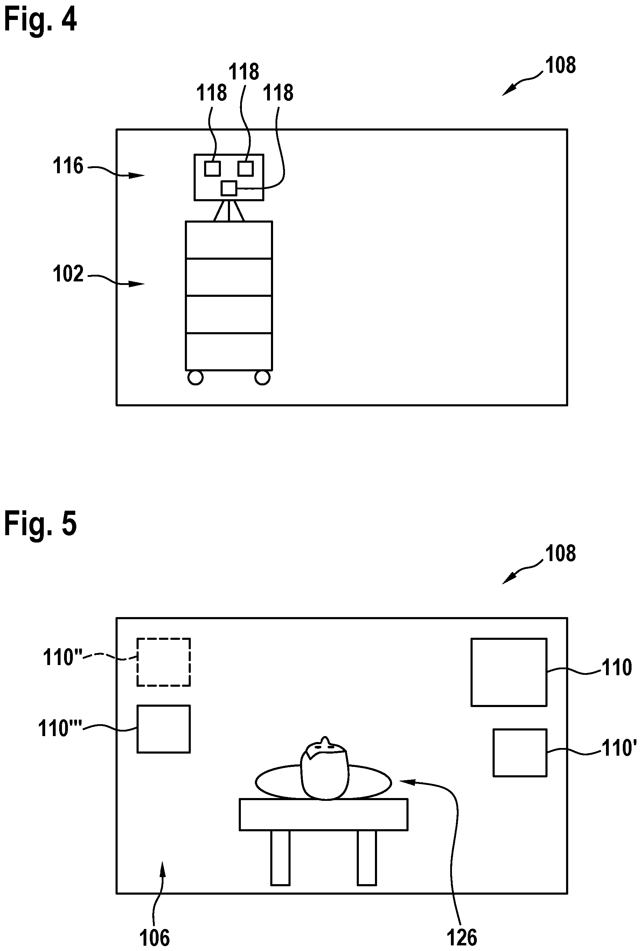

In der Fig. 4 wird wieder das Sichtfeld 108 aus der Sicht des Benutzers 124 schematisch dargestellt. Nun blickt der Benutzer 124 jedoch in Richtung der Patientenüberwachungseinheit 102, die die Anzeigevorrichtung 116 umfasst, auf der Vitalinformationsdarstellungen 118 angezeigt werden. Dies wird von der Sichtfelderkennungseinheit 112 erkannt. Die Vitalinformationsdarstellungen 118 geben zumindest im Wesentlichen dieselbe Information wieder, wie die Vitalinformationseinblendungen 110, 110`, 110" gemäß Fig. 3. Folglich wird von einer Einblendung von Vitalinformationseinblendungen abgesehen.

In der Fig. 5 wird wieder dieselbe Sicht des Benutzer 124 auf die Umgebung 106 wie in der Fig. 3 gezeigt. Im Sichtfeld 108 ist also wieder der Patient 126 angeordnet. Allerdings wurde mittels der Patientenzustandserkennungseinheit 140 ein kritischer Vitalzustand des Patienten 126 erkannt. Um den Benutzer 124 darauf aufmerksam zu machen und um wichtige Information über Vitalparameter hervorzuheben, wird die Vitalinformationseinblendung 110 in Abhängigkeit eines ihr zugrunde liegenden Patientenzustandsparameters zu vergrößert. Die Vitalinformationseinblendung 110' wird unverändert vorgenommen, da sie nach wie vor grundsätzlich wichtig ist, jedoch im Vergleich zur Vitalinformationseinblendung 110' nachrangig wichtig ist. Die Vitalinformationseinblendung 110" wird in Abhängigkeit des Patientenzustandsparameters blinkend vorgenommen. Denkbar ist auch eine visuell verstärkte Einblendung, etwa indem der Kontrast erhöht wird und/oder die Farbe der Einblendung. Zusätzlich wird der Patientenzustandsparameters 110‴ eingeblendet, damit der Benutzer 124 zusätzliche wichtige Information über den Vitalzustand erhält. Die Vitalinformationseinblendungen 110, 110`, 110", 110‴ sind gegeneinander abgestuft für den Benutzer 124 visuell wahrnehmbar. Die Abstufung wird anhand der Wichtigkeit der den Vitalinformationseinblendung 110, 110`, 110", 110‴ zugrunde liegenden Vitalparameter vorgenommen.

In der Fig. 6 ist das medizinische System 100 in einer schematischen Darstellung während des Durchführens einer Intubation gezeigt. Die Fig. 6 weist große Ähnlichkeit zu der der Fig. 1 auf. Es wird daher vorrangig auf Unterschiede eingegangen. Der Benutzer 124 trägt die tragbare Anzeige 104`, die die Sichtfelderkennungseinheit 112 und die Patientenzustandserkennungseinheit 140 umfasst. Er ist beim Durchführen einer Prozedur gezeigt. Die Prozedur ist eine Intubation. Der Benutzer 124 führt also gerade einen Tubus 121 der Luftröhre des Patienten zu. Dazu benutzt er ein Videostilett, auf das der Tubus 121 aufgesteckt ist. Das Videostilett ist ein medizinisches Bildgebungsgerät 120. Mittels des Videostiletts können Abbildungen der Luftröhre erstellt werden, damit der Tubus 121 besser platziert werden kann. Bilddaten, die mittels des Videostiletts erzeugt werden, werden kabellos an die Anzeigevorrichtung 116 übertragen. Die Anzeigevorrichtung 116 erzeugt basierend auf den Bilddaten eine Darstellung 122 für den Benutzer. Der Benutzer kann also auf die Anzeigevorrichtung 116 blicken, um etwa eine Position des Tubus 121 innerhalb der Luftröhre abzuschätzen. Mittels der Prozedurschritterkennungseinheit 150 wird einer von mehreren Schritten zur Durchführung der Intubation erkannt und ein entsprechender Prozedurschrittparameter erstellt. Anhand dieses Parameters erzeugt die tragbare Anzeige 104` eine für den entsprechenden Schritt wichtige

Vitalinformationseinblendung. In unterschiedlichen Schritten sind unterschiedliche Vitalparameter wichtig. Nachfolgend ist eine beispielhafte Auflistung von Schritten zur Durchführung der Intubation angegeben. Zu jedem Schritt werden wichtige Vitalparameter genannt, auf denen zumindest eine Vitalinformationseinblendung beruht.
1. Vorbereitung des Patienten:
   Wichtige Vitalparameter: Keine spezifischen Vitalparameter in diesem Schritt. Es wird also keine Einblendung vorgenommen.
2. Einführen des Videostiletts:
   Wichtige Vitalparameter: Herzfrequenz, Blutdruck.
3. Identifikation des Kehlkopfs:
   Wichtige Vitalparameter: Herzfrequenz, Blutdruck.
4. Einführen des Tubus 121:
   Wichtige Vitalparameter: Herzfrequenz, Blutdruck, Sauerstoffsättigung.
5. Sicherung des Tubus 121:
   Wichtige Vitalparameter: Sauerstoffsättigung, Kohlendioxid in den Atemgasen.
6. Abschluss und Kontrolle:
   Wichtige Vitalparameter: Herzfrequenz Blutdruck, Sauerstoffsättigung, Kohlendioxid in den Atemgasen.

Insbesondere bei dem Abschluss und der Kontrolle kann der Benutzer 124 folglich auf den Patienten 126 blicken und bekommt die entsprechend wichtigen Vitalinformationseinblendungen eingeblendet. Dies geschieht automatisch, indem der entsprechende Schritt erkannt wird.

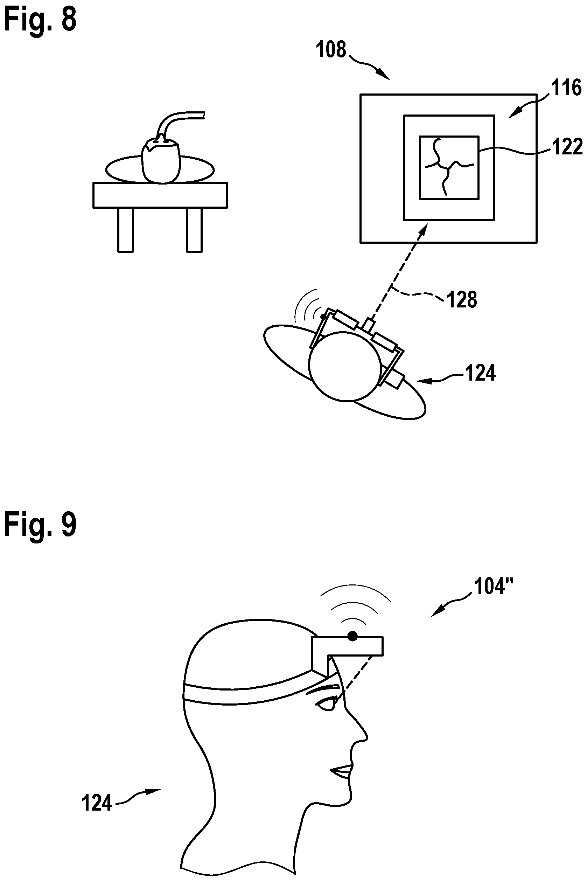

Zusätzlich zu dem Prozedurschrittparameter kann basierend auf dem Sichtfeldparameter die tragbare Anzeige 104` die Einblendung der Vitalinformationseinblendung vornehmen oder unterlassen. Dazu sei auf die Fig. 7 und 8 verwiesen.

Blickt der Benutzer 124 in Richtung des Patienten 126, an dem er die Intubation durchführt mittels der Bildgebungsvorrichtung 120 und des Tubus 121, sodass diese in seinem Sichtfeld 108 sind, wird zumindest eine Vitalinformationseinblendung 110 vorgenommen. Eine zweite Vitalinformationseinblendung 110' kann vorgenommen werden, wenn dies entsprechend dem Prozedurschrittparameter vorgesehen ist. Die Anzeigevorrichtung 116, auf der die Darstellung 122 angezeigt wird, ist nicht in seinem Sichtfeld. Dies wird unter anderem anhand der Ermittlung der Blickrichtung 128 erkannt.

Wendet der Benutzer 124 hingegen seinen Blick der Anzeigevorrichtung 116 zu, wie in der Fig. 8 gezeigt, wird eine Einblendung einer Vitalinformationseinblendung unterlassen. Unter anderem anhand der Blickrichtung 128 wird erkannt, dass die Anzeigevorrichtung 116 und die Darstellung 122 in seinem Sichtfeld 108 ist.

In der Fig. 9 ist eine weitere Ausführungsform einer tragbaren Anzeige 104" in einer schematischen Darstellung gezeigt. Die tragbare Anzeige 104" ist ein Retina-Projektor, der dazu eingerichtet ist, eine Darstellung und/oder eine Vitalinformationseinblendung auf die Retina des Benutzers 124 zu projizieren. Die tragbare Anzeige 104" kann an einem Stirnband befestigt sein, sodass sie am Kopf des Benutzers 124 tragbar ist. Alternativ oder zusätzlich kann die tragbare Anzeige 104" an einer optischen Brille des Benutzers befestigt werden. Die tragbare Anzeige 104" kann zumindest im Wesentlichen dieselbe Funktionalität und/oder dieselben Merkmale aufweisen wie die Anzeigen 104, 104`.

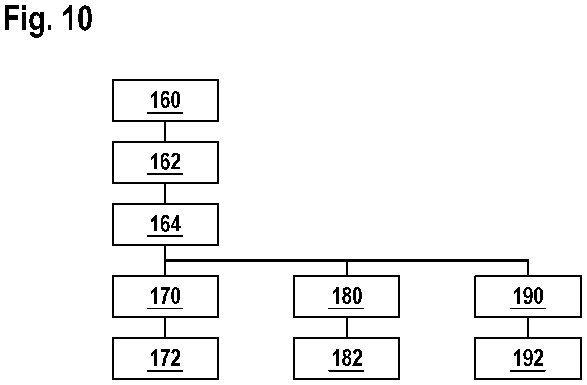

Die Fig. 10 zeigt eine schematisches Ablaufdiagramm eines Verfahrens, insbesondere durchgeführt mittels eines medizinischen Systems 100 nach zumindest einem der hierin beschriebenen Aspekte und/oder einer tragbaren Anzeige 104, 104`, 104" nach einem der hierin beschriebenen Aspekte.

Das Verfahren umfasst den Schritt 160 eines Erfassens eines Vitalparameters eines Patienten 126, den Schritt 162 eines Bereitstellens einer tragbaren Anzeige 104, 104`, 104" für einen Benutzer 124, die an einem Kopf eines Benutzers 124 tragbar ist und die in einem getragenen Zustand dem Benutzer 124 eine zumindest im Wesentlichen unbeeinträchtigte Betrachtung einer Umgebung 106 in einem Sichtfeld 108 des Benutzers 124 gestattet, und den Schritt 164 eines Einblendens einer Vitalinformationseinblendung 110 in dem Sichtfeld 108 des Benutzers 124 mittels der tragbaren Anzeige 104 in einem getragenen Zustand der Anzeige 104, die auf dem Vitalparameter beruht. Je nachdem, welche Erkennungseinheiten 112, 140, 150 vorgesehen sind, umfasst das Verfahren zumindest einen Schritt 170 eines Ermittelns eines Sichtfeldparameters, der sich auf ein aktuelles Sichtfeld 108 des Benutzers 124 bezieht, den Schritt 172 eines Vornehmens oder Unterlassens des Einblendens der Vitalinformationseinblendung 110 in Abhängigkeit des Sichtfeldparameters, den Schritt 180 eines Ermittelns eines Patientenzustandsparameters, der sich auf einen aktuellen Vitalzustand des Patienten 126 bezieht, den Schritt 182 eines Vornehmens oder Unterlassens des Einblendens der Vitalinformationseinblendung 110 in Abhängigkeit des Patientenzustandsparameters, den Schritt 190 eines Ermittelns eines Prozedurschrittparameters, der beschreibt, in welchem Schritt von mehreren Schritten einer vom Benutzer 124 am Patienten 126 durchzuführenden Prozedur sich der Benutzer 124 aktuell befindet, und/oder den Schritt 192 eines Vornehmens oder Unterlassens des Einblendens der Vitalinformationseinblendung 110 in Abhängigkeit des Prozedurschrittparameters.

### Bezugszeichenliste

- 100: Medizinisches System
- 102: Patientenüberwachungseinheit
- 104: tragbare Anzeige
- 106: Umgebung
- 108: Sichtfeld
- 110: Vitalinformationseinblendung
- 112: Sichtfelderkennungseinheit
- 114: Inhalt
- 116: Anzeigevorrichtung
- 118: Vitalinformationsdarstellung
- 120: medizinisches Bildgebungsinstrument
- 121: Tubus
- 122: Darstellung
- 124: Benutzer
- 126: Patient
- 127: Behandlungsliege
- 128: Blickrichtung
- 130: optische Korrekturbrille
- 132: Alarmvorrichtung
- 134: Kamera
- 140: Patientenzustandserkennungseinheit
- 150: Prozedurschritterkennungseinheit

### Die Offenbarung betrifft unter anderem die folgenden Aspekte:

Aspekt 1. Medizinisches System (100) umfassend:
   eine Patientenüberwachungseinheit (102), die dazu eingerichtet ist, einen Vitalparameter eines Patienten (126) zu erfassen;
   eine tragbare Anzeige (104), die an einem Kopf eines Benutzers (124) tragbar ist und die in einem getragenen Zustand dem Benutzer (124) eine zumindest im Wesentlichen unbeeinträchtigte Betrachtung einer Umgebung (106) in einem Sichtfeld (108) des Benutzers (124) gestattet, wobei die tragbare Anzeige (104) dazu eingerichtet ist, in dem getragenen Zustand eine Vitalinformationseinblendung (110) in dem Sichtfeld (108) des Benutzers (124) einzublenden, die auf dem Vitalparameter beruht; und
   eine Sichtfelderkennungseinheit (112), die dazu eingerichtet ist, zumindest einen Sichtfeldparameter zu ermitteln, der sich auf ein aktuelles Sichtfeld (108) des Benutzers (124) bezieht;
   wobei die tragbare Anzeige (104) dazu eingerichtet ist, eine Einblendung der Vitalinformationseinblendung (110) in Abhängigkeit des Sichtfeldparameters vorzunehmen oder zu unterlassen.
Aspekt 2. Medizinisches System (100) nach Aspekt 1,
   wobei der Sichtfeldparameter Information bezüglich des Inhalts (114) des Sichtfelds (108) des Benutzers (124) umfasst.
Aspekt 3. Medizinisches System (100) nach Aspekt 2,
   ferner umfassend eine Anzeigevorrichtung (116), die dazu eingerichtet ist, eine Vitalinformationsdarstellung (118) anzuzeigen, die auf dem Vitalparameter beruht,
   wobei die tragbare Anzeige (104) dazu eingerichtet ist, die Einblendung der Vitalinformationseinblendung (110) zu unterlassen, wenn der Sichtfeldparameter anzeigt, dass sich die Anzeigevorrichtung (116) in dem Sichtfeld (108) des Benutzers (124) befindet.
Aspekt 4. Medizinisches System (100) nach einem der vorhergehenden Aspekte,
   ferner umfassend:
   ein medizinisches Bildgebungsinstrument (120), das dazu eingerichtet ist, Bilddaten zu erzeugen und die Bilddaten zu übertragen;
   eine Anzeigevorrichtung (116), die dazu eingerichtet ist, die Bilddaten zu empfangen und eine Darstellung (122) für einen Benutzer (124) zu erzeugen;
   wobei die tragbare Anzeige (104) dazu eingerichtet ist, die Einblendung der Vitalinformationseinblendung (110) zu unterlassen, wenn der Sichtfeldparameter anzeigt, dass sich die Anzeigevorrichtung (116) in dem Sichtfeld (108) des Benutzers (124) befindet.
Aspekt 5. Medizinisches System (100) nach einem der vorhergehenden Aspekte,
   wobei die tragbare Anzeige (104) dazu eingerichtet ist, die Einblendung der Vitalinformationseinblendung (110) vorzunehmen, wenn der Sichtfeldparameter anzeigt, das sich der Patient (126) in dem Sichtfeld (108) des Benutzers (124) befindet.
Aspekt 6. Medizinisches System (100) nach einem der vorhergehenden Aspekte,
   wobei die Sichtfelderkennungseinheit (112) an der tragbaren Anzeige (104) angeordnet ist.
Aspekt 7. Medizinisches System (100) nach einem der vorhergehenden Aspekte,
   wobei die Sichtfelderkennungseinheit (112) separat von der tragbaren Anzeige (104) angeordnet ist.
Aspekt 8. Medizinisches System (100) nach einem der vorhergehenden Aspekte,
   wobei die Sichtfelderkennungseinheit (112) dazu eingerichtet ist, die Blickrichtung (128) des Benutzers (124) zu ermitteln; und
   wobei der Sichtfeldparameter auf der ermittelten Blickrichtung (128) beruht.
Aspekt 9. Medizinisches System (100) nach einem der vorhergehenden Aspekte,
   wobei die tragbare Anzeige (104) in eine optische Korrekturbrille (130) integrierbar ist.
Aspekt 10. Medizinisches System (100) nach einem der vorhergehenden Aspekte,
   wobei die tragbare Anzeige (104) eine Alarmvorrichtung (132) umfasst, die dazu eingerichtet ist, den Benutzer (124) zu alarmieren, wenn zumindest einer der Vitalparameter in einem kritischen Bereich ist.
Aspekt 11. Medizinisches System (100) nach einem der vorhergehenden Aspekte,
   wobei die tragbare Anzeige (104) dazu eingerichtet ist, die Einblendung der Vitalinformationseinblendung (110) unabhängig von dem Sichtfeldparameter vorzunehmen, wenn zumindest einer der Vitalparameter in einem kritischen Bereich ist.
Aspekt 12. Tragbare Anzeige (104) für ein Medizinisches System (100) nach einem der vorhergehenden Aspekte.
Aspekt 13. Tragbare Anzeige (104), insbesondere für ein Medizinisches System (100) nach einem der Aspekte 1 bis 11, die an einem Kopf eines Benutzers (124) tragbar ist und die in einem getragenen Zustand dem Benutzer (124) eine zumindest im Wesentlichen unbeeinträchtigte Betrachtung einer Umgebung (106) in einem Sichtfeld (108) des Benutzers (124) gestattet, wobei die tragbare Anzeige (104) dazu eingerichtet ist, in dem getragenen Zustand eine Vitalinformationseinblendung (110) in dem Sichtfeld (108) des Benutzers (124) einzublenden, die auf einem Vitalparameter beruht; und
   eine Sichtfelderkennungseinheit (112) umfasst, die dazu eingerichtet ist, zumindest einen Sichtfeldparameter zu ermitteln, der sich auf das aktuelle Sichtfeld (108) des Benutzers (124) bezieht;
   wobei die tragbare Anzeige (104) dazu eingerichtet ist, eine Einblendung der Vitalinformationseinblendung (110) in Abhängigkeit des Sichtfeldparameters vorzunehmen und/oder zu unterlassen.
Aspekt 14. Verfahren zum Betrieb eines medizinischen Systems (100) nach einem der Aspekte 1 bis 11 und/oder einer tragbaren Anzeige (104) nach Aspekt 12 oder 13.
Aspekt 15. Verfahren, insbesondere durchgeführt mittels eines medizinischen Systems (100) nach einem der Aspekte 1 bis 11 und/oder einer tragbaren Anzeige (104) nach Aspekt 12 oder 13, umfassend die Schritte:
   Erfassen eines Vitalparameters eines Patienten (126);
   Bereitstellen einer tragbaren Anzeige (104) für einen Benutzer (124), die an einem Kopf eines Benutzers (124) tragbar ist und die in einem getragenen Zustand dem Benutzer (124) eine zumindest im Wesentlichen unbeeinträchtigte Betrachtung einer Umgebung (106) in einem Sichtfeld (108) des Benutzers (124) gestattet;
   Einblenden einer Vitalinformationseinblendung (110) in dem Sichtfeld (108) des Benutzers (124) mittels der tragbaren Anzeige (104) in einem getragenen Zustand der Anzeige (104), die auf dem Vitalparameter beruht;
   Ermitteln eines Sichtfeldparameters, der sich auf ein aktuelles Sichtfeld (108) des Benutzers (124) bezieht; und
   Vornehmen oder Unterlassen des Einblendens der Vitalinformationseinblendung (110) in Abhängigkeit des Sichtfeldparameters.
Aspekt 16. Medizinisches System (100) umfassend:
   eine Patientenüberwachungseinheit (102), die dazu eingerichtet ist, einen Vitalparameter eines Patienten (126) zu erfassen;
   eine tragbare Anzeige (104), die an einem Kopf eines Benutzers (124) tragbar ist und die in einem getragenen Zustand einem Benutzer (124) eine zumindest im Wesentlichen unbeeinträchtigte Betrachtung einer Umgebung (106) in einem Sichtfeld (108) des Benutzers (124) gestattet, wobei die tragbare Anzeige (104) dazu eingerichtet ist, in dem getragenen Zustand eine Vitalinformationseinblendung (110) in dem Sichtfeld (108) des Benutzers (124) einzublenden, die auf dem einen Vitalparameter beruht; und
   eine Prozedurschritterkennungseinheit (150), die dazu eingerichtet ist, zumindest einen Prozedurschrittparameter zu ermitteln, der beschreibt, in welchem Schritt von mehreren Schritten einer vom Benutzer (124) am Patienten (126) durchzuführenden Prozedur sich der Benutzer (124) aktuell befindet;
   wobei die tragbare Anzeige (104) dazu eingerichtet ist, eine Einblendung der Vitalinformationseinblendung (110) in Abhängigkeit des Prozedurschrittparameters vorzunehmen und/oder zu unterlassen.
Aspekt 17. Medizinisches System (100) nach Aspekt 16,
   wobei die Patientenüberwachungseinheit (102) dazu eingerichtet ist, unterschiedliche Vitalparameter zu erfassen;
   wobei die tragbare Anzeige (104) dazu eingerichtet ist, in dem getragenen Zustand mehrere unterschiedliche Vitalinformationseinblendungen (110) in dem Sichtfeld (108) des Benutzers (124) einzublenden, die auf unterschiedlichen Vitalparametern beruhen;
   und wobei die tragbare Anzeige (104) dazu eingerichtet ist, in Abhängigkeit des jeweiligen von der Prozedurschritterkennungseinheit (150) erkannten Schritts der durchzuführenden Prozedur jeweils zumindest eine der mehreren unterschiedlichen Vitalinformationseinblendungen (110) vorzunehmen.
Aspekt 18. Medizinisches System (100) nach Aspekt 16 oder 17,
   wobei die tragbare Anzeige (104) dazu eingerichtet ist, die Vitalinformationseinblendung (110) in Abhängigkeit des Prozedurschrittparameters visuell zu verstärken.
Aspekt 19. Medizinisches System (110) nach einem der vorhergehenden Aspekte, insbesondere nach einem der Aspekte 16 bis 18,
   wobei die tragbare Anzeige (104) dazu eingerichtet ist, die Vitalinformationseinblendung (110) in Abhängigkeit des Prozedurschrittparameters zu vergrößern.
Aspekt 20. Medizinisches System (110) nach einem der vorhergehenden Aspekte, insbesondere nach einem der Aspekte 16 bis 19,
   wobei die tragbare Anzeige (104) dazu eingerichtet ist, die Vitalinformationseinblendung (110) in Abhängigkeit des Prozedurschrittparameters zumindest teilweise blinkend vorzunehmen.
Aspekt 21. Medizinisches System (110) nach einem der vorhergehenden Aspekte, insbesondere nach einem der Aspekte 16 bis 20,
   wobei die tragbare Anzeige (104) dazu eingerichtet ist, die Vitalinformationseinblendung (110) unabhängig von einer Blickrichtung (128) des Benutzers (124) vorzunehmen.
Aspekt 22. Medizinisches System (100) nach einem der vorhergehenden Aspekte, insbesondere nach einem der Aspekte 16 bis 21,
   wobei die Prozedurschritterkennungseinheit (150) eine Kamera (134) umfasst, die dazu eingerichtet ist, Bilddaten zu erzeugen, und
   wobei die Prozedurschritterkennungseinheit (150) dazu eingerichtet ist, den zumindest einen Prozedurschrittparameter anhand der Bilddaten zu ermitteln.
Aspekt 23. Medizinisches System (100) nach Aspekt 22,
   wobei die Kamera (134) an der tragbaren Anzeige (104) angeordnet ist.
Aspekt 24. Medizinisches System (100) nach Aspekt 22,
   wobei die Kamera (134) separat von der tragbaren Anzeige (104) angeordnet ist.
Aspekt 25. Medizinisches System (100) nach einem der vorhergehenden Aspekte, insbesondere nach einem der Aspekte 16 bis 24,
   wobei die Prozedur eine Intubation des Patienten (126) ist.
Aspekt 26. Medizinisches System (100) nach einem der vorhergehenden Aspekte, insbesondere nach einem der Aspekte 16 bis 25, ferner umfassend:
   eine Patientenzustandserkennungseinheit (140), die dazu eingerichtet ist, zumindest einen Patientenzustandsparameter zu ermitteln, der sich auf einen aktuellen Vitalzustand des Patienten (126) bezieht.
Aspekt 27. Medizinisches System (100) nach Aspekt 26,
   wobei die tragbare Anzeige (104) dazu eingerichtet ist, eine Einblendung der Vitalinformationseinblendung (110) in Abhängigkeit des Prozedurschrittparameters und des Patientenzustandsparameter vorzunehmen oder zu unterlassen.
Aspekt 28. Medizinisches System (100) nach Aspekt 27,
   die tragbare Anzeige (104) dazu eingerichtet ist, dann, wenn der Patientenzustandsparameter einen kritischen Vitalzustand des Patienten (126) anzeigt, eine Einblendung der Vitalinformationseinblendung (110) unabhängig von dem Prozedurschrittparameter vorzunehmen oder zu unterlassen.
Aspekt 29. Medizinisches System (110) nach einem der vorhergehenden Aspekte, insbesondere nach einem der Aspekte 16 bis 28,
   wobei die tragbare Anzeige (104) in eine optische Korrekturbrille (130) integrierbar ist.
Aspekt 30. Tragbare Anzeige (104) für ein medizinisches System (100) nach einem der vorhergehenden Aspekte, insbesondere nach einem der Aspekte 16 bis 29.
Aspekt 31. Tragbare Anzeige (104), insbesondere für ein medizinisches System (100) nach einem der Aspekte 16 bis 29, die an einem Kopf eines Benutzers (124) tragbar ist und die in einem getragenen Zustand einem Benutzer (124) eine zumindest im Wesentlichen unbeeinträchtigte Betrachtung einer Umgebung (106) in einem Sichtfeld (108) des Benutzers (124) gestattet, wobei die tragbare Anzeige (104) dazu eingerichtet ist, in dem getragenen Zustand eine Vitalinformationseinblendung (110) in dem Sichtfeld (108) des Benutzers (124) einzublenden, die auf dem einen Vitalparameter beruht; und
   eine Prozedurschritterkennungseinheit (150) umfasst, die dazu eingerichtet ist, zumindest einen Prozedurschrittparameter zu ermitteln, der beschreibt, in welchem Schritt von mehreren Schritten einer vom Benutzer (124) am Patienten (126) durchzuführenden Prozedur sich der Benutzer (124) aktuell befindet;
   wobei die tragbare Anzeige (104) dazu eingerichtet ist, eine Einblendung der Vitalinformationseinblendung (110) in Abhängigkeit des Prozedurschrittparameters vorzunehmen und/oder zu unterlassen.
Aspekt 32. Verfahren zum Betrieb eines medizinischen Systems (100) nach einem der Aspekte 16 bis 29 und/oder einer tragbaren Anzeige (104) nach Aspekt 30 oder 31.
Aspekt 33. Verfahren, insbesondere durchgeführt mittels eines medizinischen Systems (100) nach einem der Aspekte 16 bis 29 und/oder einer tragbaren Anzeige (104) nach Aspekt 30 oder 31, umfassend die Schritte:
   Erfassen eines Vitalparameters eines Patienten (126);
   Bereitstellen einer tragbaren Anzeige (104) für einen Benutzer (124), die an einem Kopf eines Benutzers (124) tragbar ist und die in einem getragenen Zustand dem Benutzer (124) eine zumindest im Wesentlichen unbeeinträchtigte Betrachtung einer Umgebung (106) in einem Sichtfeld (108) des Benutzers (124) gestattet;
   Einblenden einer Vitalinformationseinblendung (110) in dem Sichtfeld (108) des Benutzers (124) mittels der tragbaren Anzeige (104) in einem getragenen Zustand der Anzeige (104), die auf dem Vitalparameter beruht;
   Ermitteln eines Prozedurschrittparameters, der beschreibt, in welchem Schritt von mehreren Schritten einer vom Benutzer (124) am Patienten (126) durchzuführenden Prozedur sich der Benutzer (124) aktuell befindet; und
   Vornehmen oder Unterlassen des Einblendens der Vitalinformationseinblendung (110) in Abhängigkeit des Prozedurschrittparameters.

## Patentansprüche

1. Medizinisches System (100) umfassend:
eine Patientenüberwachungseinheit (102), die dazu eingerichtet ist, einen Vitalparameter eines Patienten (126) zu erfassen;
eine tragbare Anzeige (104), die an einem Kopf eines Benutzers (124) tragbar ist und die in einem getragenen Zustand einem Benutzer (124) eine zumindest im Wesentlichen unbeeinträchtigte Betrachtung einer Umgebung (106) in einem Sichtfeld (108) des Benutzers (124) gestattet, wobei die tragbare Anzeige (104) dazu eingerichtet ist, in dem getragenen Zustand eine Vitalinformationseinblendung (110) in dem Sichtfeld (108) des Benutzers (124) einzublenden, die auf dem einen Vitalparameter beruht; und
eine Patientenzustandserkennungseinheit (140), die dazu eingerichtet ist, zumindest einen Patientenzustandsparameter zu ermitteln, der sich auf einen aktuellen Vitalzustand des Patienten (126) bezieht;
wobei die tragbare Anzeige (104) dazu eingerichtet ist, eine Einblendung der Vitalinformationseinblendung (110) in Abhängigkeit des Patientenzustandsparameters vorzunehmen oder zu unterlassen.

2. Medizinisches System (100) nach Anspruch 1,
wobei die tragbare Anzeige (104) dazu eingerichtet ist, die Einblendung der Vitalinformationseinblendung (110) vorzunehmen, wenn der zumindest eine Patientenzustandsparameter in einem kritischen Bereich ist, und
wobei die tragbare Anzeige (104) dazu eingerichtet ist, die Einblendung der Vitalinformationseinblendung (110) zu unterlassen, wenn der zumindest eine Patientenzustandsparameter nicht in dem kritischen Bereich ist.

3. Medizinisches System (100) nach Anspruch 1 oder 2,
wobei die tragbare Anzeige (104) eine Alarmvorrichtung (132) umfasst, die dazu eingerichtet ist, den Benutzer (124) zu alarmieren, wenn der zumindest eine Patientenzustandsparameters in einem kritischen Bereich ist.

4. Medizinisches System (100) nach Anspruch 3,
wobei die tragbare Anzeige (104) dazu eingerichtet ist, den Benutzer (124) mittels eines Vibrationsalarm zu alarmieren.

5. Medizinisches System (100) nach Anspruch 3 oder 4,
wobei die tragbare Anzeige (104) dazu eingerichtet ist, den Benutzer (124) mittels eines akustischen Alarmsignals zu alarmieren.

6. Medizinisches System (100) nach einem der vorhergehenden Ansprüche,
wobei die tragbare Anzeige (104) dazu eingerichtet ist, die Vitalinformationseinblendung (110) in Abhängigkeit des Patientenzustandsparameters visuell zu verstärken.

7. Medizinisches System (100) nach einem der vorhergehenden Ansprüche,
wobei die tragbare Anzeige (104) dazu eingerichtet ist, die Vitalinformationseinblendung (110) in Abhängigkeit des Patientenzustandsparameters zu vergrößern.

8. Medizinisches System (100) nach einem der vorhergehenden Ansprüche,
wobei die tragbare Anzeige (104) dazu eingerichtet ist, die Vitalinformationseinblendung (110) in Abhängigkeit des Patientenzustandsparameters zumindest teilweise blinkend vorzunehmen.

9. Medizinisches System (100) nach einem der vorhergehenden Ansprüche,
wobei die tragbare Anzeige (104) dazu eingerichtet ist, die Vitalinformationseinblendung (110) unabhängig von einer Blickrichtung (128) des Benutzers (124) vorzunehmen.

10. Medizinisches System (100) nach einem der vorhergehenden Ansprüche,
wobei die tragbare Anzeige (104) in eine optische Korrekturbrille (130) integrierbar ist.

11. Tragbare Anzeige (104) für ein medizinisches System (100) nach einem der vorhergehenden Ansprüche.

12. Tragbare Anzeige (104), insbesondere für ein medizinisches System (100) nach einem der Ansprüche 1 bis 10, die an einem Kopf eines Benutzers (124) tragbar ist und die in einem getragenen Zustand einem Benutzer (124) eine zumindest im Wesentlichen unbeeinträchtigte Betrachtung einer Umgebung (106) in einem Sichtfeld (108) des Benutzers (124) gestattet, wobei die tragbare Anzeige (104) dazu eingerichtet ist, in dem getragenen Zustand eine Vitalinformationseinblendung (110) in dem Sichtfeld (108) des Benutzers (124) einzublenden, die auf dem einen Vitalparameter beruht; und
eine Patientenzustandserkennungseinheit (140) umfasst, die dazu eingerichtet ist, zumindest einen Patientenzustandsparameter zu ermitteln, der sich auf einen aktuellen Vitalzustand des Patienten (126) bezieht;
wobei die tragbare Anzeige (104) dazu eingerichtet ist, eine Einblendung der Vitalinformationseinblendung (110) in Abhängigkeit des Patientenzustandsparameters vorzunehmen oder zu unterlassen.

13. Verfahren zum Betrieb eines medizinischen Systems (100) nach einem der Ansprüche 1 bis 10 und/oder einer tragbaren Anzeige (104) nach Anspruch 11 oder 12.

14. Verfahren, insbesondere durchgeführt mittels eines medizinischen Systems (100) nach einem der Ansprüche 1 bis 10 und/oder einer tragbaren Anzeige (104) nach Anspruch 11 oder 12, umfassend die Schritte:
Erfassen eines Vitalparameters eines Patienten (126);
Bereitstellen einer tragbaren Anzeige (104) für einen Benutzer (124), die an einem Kopf eines Benutzers (124) tragbar ist und die in einem getragenen Zustand dem Benutzer (124) eine zumindest im Wesentlichen unbeeinträchtigte Betrachtung einer Umgebung (106) in einem Sichtfeld (108) des Benutzers (124) gestattet;
Einblenden einer Vitalinformationseinblendung (110) in dem Sichtfeld (108) des Benutzers (124) mittels der tragbaren Anzeige (104) in einem getragenen Zustand der Anzeige (104), die auf dem Vitalparameter beruht;
Ermitteln eines Patientenzustandsparameters, der sich auf einen aktuellen Vitalzustand des Patienten (126) bezieht; und
Vornehmen oder Unterlassen des Einblendens der Vitalinformationseinblendung (110) in Abhängigkeit des Patientenzustandsparameters.
